# EUROPEAN PATENT APPLICATION

(11) **EP 2 727 944 A1**
(43) Date of publication of application: **07.05.2014**
(21) Application number: 12190645.7
(22) Date of filing: 30.10.2012
(51) Int. Cl.: C07K 16/28, A61K 39/395

(54) **Depletion of CCR2-positive monocytes for the treatment of inflammatory conditions in the gastrointestinal system**

(71) Applicant: Universitätsklinikum Regensburg, 93053 Regensburg (DE); Yeda Research and Development Co. Ltd., 76100 Rechovot (IL); Medical Research, Infrastructure and Health Services Fund at Tel Aviv Sourasky Medical Center, 64239 Tel Aviv (IL)
(72) Inventor: The designation of the inventor has not yet been filed
(74) Representative: Vossius & Partner

(57) **Abstract**

The present invention relates to an agent capable of binding to the C-C chemokine receptor type 2 (CCR2) and inducing the depletion of CCR2-positive monocytes in a subject for use in the treatment and/or prevention of an inflammatory disease of the gastrointestinal system. The present invention further relates to a method of treating and/or preventing an inflammatory disease of the gastrointestinal system comprising administering a pharmaceutically effective amount of an agent capable of binding to CCR2 and inducing the depletion of CCR2-positive monocytes in a subject to a subject in need thereof. Furthermore, the present invention relates to a pharmaceutical composition comprising the agent of the invention, and optionally a pharmaceutically acceptable carrier.

## Description

The present invention relates to an agent capable of binding to the C-C chemokine receptor type 2 (CCR2) and inducing the depletion of CCR2-positive monocytes in a subject for use in the treatment and/or prevention of an inflammatory disease of the gastrointestinal system. The present invention further relates to a method of treating and/or preventing an inflammatory disease of the gastrointestinal system comprising administering a pharmaceutically effective amount of an agent capable of binding to CCR2 and inducing the depletion of CCR2-positive monocytes in a subject to a subject in need thereof. Furthermore, the present invention relates to a pharmaceutical composition comprising the agent of the invention, and optionally a pharmaceutically acceptable carrier.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The gastrointestinal tract hosts a myriad of commensal bacteria that form a critical microbial metagenome assisting its host in nutrition. A monolayer of epithelial cells that separates the gut lumen from deeper tissue and immune cells residing in the lamina propria ensures efficient food uptake. Yet, this scenario poses a unique challenge, as the organism has to tolerate the foreign, though beneficial symbionts and constant exposure to their products. Moreover, rather, than remaining unresponsive, the host actively controls the microbiota composition by secretion of anti-microbial peptides and immunoglobulins (Bevins and Salzman, 2011; Peterson et al., 2007). Conversely, defined intestinal commensals, such as *Clostridiae*, have recently been shown to critically shape the gut associated immune system, e.g. the prevalence of distinct helper and regulatory T cell populations (Atarashi et al., 2011; lvanov et al., 2009). While actively engaging the commensal microbial challenge, the organism has to remain sensitive to deviations from this "primed homeostasis" and rapidly respond to invading entero-pathogens or injuries causing epithelial damage. Failure to maintain this exquisite balance and hyper-responsiveness in genetically predisposed individuals is considered to be associated with the development of chronic inflammatory bowel disorders (IBD), such as Crohn's disease and Ulcerative colitis (Cho, 2008).

Inflammatory bowel diseases are a group of idiopathic, chronic relapsing inflammatory intestinal conditions characterized by abdominal pain, diarrhea, bleeding and mal-absorption. The two main IBD subtypes are Crohn's disease (CD) and ulcerative colitis (UC). CD generally involves the ileum and colon, but it can affect any region of the intestine, often discontinuously and is associated with transmural inflammation and pathologic manifestations such as granulomas, strictures and fistulas. UC, on the other hand, involves the rectum and may affect part of the colon or the entire colon (pancolitis) in an uninterrupted pattern and the inflammation is typically confined to the mucosa. Their etiology is not clear but in general, IBD is suggested to be caused by an inappropriate inflammatory response to intestinal microbes in a genetically susceptible host. IBD usually manifests in the second or third decade of life, and effects about 1.4 million people in the USA, 2.2 million people in Europe, and with an alarming rise in previous low-incidence areas, such as Asia. Patients with IBD are at high risk for primary sclerosing cholangitis, ankylosing spondylitis, psoriasis and for colon cancer.

Treatment of IBD includes lifestyle alterations (e. g. smoking cessation for patients with Crohn's disease and restrictive diet habits), medical management and surgical interventions. Medical efforts today are directed towards suppressing the abnormal inflammatory response. A seminal therapeutic advance was the introduction of treatment with an anti-TNF-α monoclonal antibody, which is particularly effective in CD: However, anti-TNFα therapy is often limited by a partial effectiveness (only 65 % of IBD patients) and loss of efficacy as well as high propensity for an infection or reactivation of infections such as tuberculosis. Moreover, recent studies have demonstrated that TNFα suppresses acute intestinal inflammation by promoting local steroidogenesis and absence of TNF results in a lack of colonic glucocorticoid synthesis and exacerbation of experimental colitis implying that extensive anti-TNFα treatment may be deleterious rather than beneficial for the treatment of IBD. Collectively, the current lack of effective therapy for IBD and its increasing prevalence underscores the need for novel therapeutic strategies.

Tissue resident dendritic cells (DCs) and macrophages (MΦs) are key players in the control of innate and adaptive immune reactions. Historically categorized by their anatomic locations and surface marker profiles, DC and MΦ definitions have more recently been extended to include differential growth and transcription factor requirements, subset-specific gene expression signatures and distinct ontogenies (Hashimoto et al., 2011). Moreover, emerging evidence for unique contributions of DCs and MΦs to the initiation and control of intestinal innate and adaptive immune responses has lend support to the notion of distinct functional entities (Varol et al., 2010).

The intestinal steady state mononuclear phagocyte compartment comprises two types of preDC-derived, Flt3L-dependent CD11c⁺ dendritic cells that are in the gut marked by expression of the alphaE integrin CD103. Like in lymphoid organs, these classical dendritic cells can be further subdivided according to their dependence on the transcription factor BatF3 into CD11b⁻ and CD11b⁺ subpopulations (Edelson et al., 2010). BatF3-dependent CD103⁺ CD11b⁻ dendritic cells are considered to reside in gut-associated lymphoid tissue, such as isolated lymphoid follicles (ILFs) (Bogunovic et al., 2009). CD103⁺ CD11b⁺ dendritic cells, on the other hand, populate especially the small bowel lamina propria, where as sentinels they can capture antigen and migrate in a CCR7-dependent manner to the draining lymph nodes (LNs) to prime T cell responses (Jaensson et al., 2008; Johansson-Lindbom et al., 2005; Sun et al., 2007; Worbs et al., 2006).

In addition to these short-lived dendritic cells, the lamina propria harbors a major population of resident non-migratory CD11c^{lo} MΦs, which are marked by prominent surface expression of F4/80 (EMR1) and the CX₃CR1 chemokine receptor. CX₃CR1^{hi} MΦs are derived from monocytes in an M-CSF dependent pathway (Bogunovic et al., 2009; Varol et al., 2009) and acquire in the gut, potentially under the influence of the microflora-exposed epithelium a distinct non-inflammatory gene expression profile (Rivollier et al., 2012). CX₃CR1^{hi} MΦs have an extended half-life and are considered to critically contribute to the maintenance or local expansion of T effector or regulatory cells in the lamina propria (Hadis et al., 2011). They have been shown to gain access to the gut lumen by virtue of trans-epithelial dendrites (Niess et al., 2005; Rescigno et al., 2001) and are believed to contribute to gut homeostasis by production of IL10 (Denning et al., 2007; Hadis et al., 2011).

Monocytes, the third population of mononuclear phagocytes, are continuously generated in the bone marrow (BM) from dedicated MΦ/DC precursors (MDPs) (Varol et al., 2007) and released to the circulation. Discrete expression levels of CX₃CR1/GFP in CX₃CR1^{gfp} animals led to the identification of two monocyte subsets in mice as CX₃CR1^{int} Ly6C^{hi} and CX₃CR1^{hi} Ly6C^{lo} cells (Geissmann et al., 2003; Palframan et al., 2001). CX₃CR1^{hi} Ly6C^{lo} "patrolling" monocytes - the correlate of human CD14^{dim} CD16⁺ cells (Cros et al., 2010) - have been shown to adhere and crawl along the luminal vessel surfaces of endothelial cells (Auffray et al., 2007). CX₃CR1^{int} Ly6C^{hi} monocytes, the correlate of human CD14⁺ CD16⁺ and CD14⁺ CD16⁻ monocytes (Cros et al., 2010), are poised to traffic to sites of infection and inflammation (Geissmann et al., 2003; Serbina and Pamer, 2006). CX₃CR1^{int} Ly6C^{hi} monocytes have a short circulation half-life (Liu et al., 2009) and tend to differentiate in steady state by default into CX₃CR1^{hi} Ly6C^{lo} cells (Varol et al., 2007). In contrast under inflammation, CX₃CR1^{int} Ly6C^{hi} monocytes are by virtue of their expression of the chemokine receptor CCR2 efficiently recruited to the sites of insult where they can transiently complement resident MΦ populations (Ajami et al., 2011). Notably, the intestinal MΦ compartment is unique in that it continuously recruits monocytes in homeostasis for its replenishment - most likely due to tonic low-grade inflammation caused the microflora exposure of this tissue. In addition to the MΦ fate, Ly6C^{hi} monocytes have been shown to give rise to a distinct population of TNF/iNOS-producing inflammatory monocyte-derived dendritic cells, as well as cells that share other functional features of classical dendritic cells, although functional contributions of these cells remain less well defined (Cheong et al., 2010; Serbina et al., 2003).

Despite the fact that a lot of effort has been invested into identifying the key players involved in maintaining the balance between tolerance towards commensal bacteria and responses to invading entero-pathogens or injuries causing epithelial damage, there is still a need to identify satisfying methods for the treatment or towards the development of a treatment of inflammatory conditions in the gastrointestinal system, such as chronic inflammatory bowel disorders including, amongst others, Crohn's disease and Ulcerative colitis.

This need is addressed by the provision of the embodiments characterized in the claims.

Accordingly, the present invention relates to an agent capable of binding to the C-C chemokine receptor type 2 (CCR2) and inducing the depletion of CCR2-positive monocytes in a subject for use in the treatment and/or prevention of an inflammatory disease of the gastrointestinal system.

The agent for use in accordance with the present invention is "an agent capable of binding to the C-C chemokine receptor type 2 (CCR2)".

The C-C chemokine receptor type 2 (CCR2) is known in the art. It is an integral membrane protein that belongs to the C-C chemokine receptor family that specifically binds and responds to cytokines of the C-C chemokine family. CCRs represent a subfamily of the large family of G protein-linked receptors that are known as seven transmembrane (7-TM) proteins since they span the cell membrane seven times. To date, ten members of the C-C chemokine receptor subfamily have been described. CCR2 can interact with CCL2, CCL8, CCL7, CCL13 and CCL16 and has been identified on the surface of monocytes, activated memory T cells, B cells, and basophils in humans, and also in peritoneal macrophages in mice. CCR2 is also designated CD192 and human monocyte chemoattractant protein-1 receptor and exists in two different splice variants in humans, CCR2A and CCR2B (Wong et al. (1997) J Biol Chem, 272:1038-1045). The corresponding orthologs of CCR2 from several other species are also known and can easily be determined by a person skilled in the art, for example, by means of sequence searches starting from the human CCR2. It will be appreciated that such orthologs are also encompassed by the present invention, also in cases where they are not named "CCR2". For example, when the subject is mouse, the mouse CCR2 is the target for the agent capable of binding to the CCR2, when the subject is rabbit, the rabbit CCR2 is the target for the agent capable of binding to the CCR2 etc. CCR2 has been described in the art, e.g. in Charo *et al.* 1994. Human CCR2 has the sequence represented by Gene ID: 729230 while murine CCR2 has the sequence represented by Gene ID: 12772.

Agents binding to CCR2 are well known in the art and include, without being limiting, antibodies as well as small molecular substances binding to CCR2 or chemokines or modified chemokines binding to CCR2 (like for example CCL2, CCL8, CCL16, CCL7 and CCL13). As defined herein above, it is required that binding of said agent to CCR2 results in the induction of depletion of CCR2-positive monocytes. If an agent does not itself induce the depletion of CCR2-positive monocytes, but only binds to the C-C chemokine receptor type 2, then said agent may be coupled to cytotoxic agents, such as for example *Pseudomonas* exotoxin A or diphtheria toxin fragment A. Alternatively the agent might be coupled with a second reagent that induces cytotoxicity or recruits cytotoxic cells (like T cells, NK cells etc, by e.g. an antibody directed against CD3 or Fc-receptors) or binds or activates cytotoxic molecules or inactivates inhibitors of cytotoxicity (e.g. complement inhibitors). The term "depletion", in accordance with the present invention, relates to a reduction in the number of CCR2-positive monocytes in the peripheral blood of the subject by removal of said cells, e.g. by destruction thereof. Preferably, at least 20% of the CCR2-positive monocytes in the peripheral blood of the subject are depleted, more preferably at least 30%, such as e.g. at least 40%, more preferably at least 50%, such as e.g. at least 60%, at least 70%, at least 80%, even more preferably at least 90% and most preferably all, i.e. 100% of the CCR2- positive monocytes in the peripheral blood of the subject are depleted. Means and methods to test whether an agent induced the depletion of CCR2-positive monocytes are well known and include, without being limiting FACS analysis, wherein the monocytes are specifically stained via a combination of surface markers found on CCR2-positive monocytes, like CD14, CD16, and CD45 (for human monocytes) or CD11b, CD115 and Ly6C and CD45 (for mouse monocytes). The amount of CCR2-positive monocytes in the presence of the agent to be tested can thus be determined and compared to the amount of CCR2-positive monocytes in the absence of the agent. Counting beads are typically included during FACS analysis to quantify cell numbers. Alternatively, absolute leukocyte numbers are counted and the percentage of monocytes from total leukocytes is provided (Mercolino *et al*. 1995).

The term "antibody" as used in accordance with the present invention comprises polyclonal and monoclonal antibodies, as well as derivatives or fragments thereof, which still retain their binding specificity. Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies. Preferably, the antibody is a humanized antibody.

Various techniques for the production of antibodies are well known in the art and described, e.g. in Harlow and Lane (1988) and (1999), loc. cit. For example, the antibodies can be produced as peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique originally developed by Köhler and Milstein Nature 256 (1975), 495-497, the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to a target protein (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

An antibody against human CCR2 can for example be produced by immunising mice, rats, hamster, or rabbits with cells that express CCR2 on their surface, such as e.g. cells obtained from a cell line transfected with CCR2. Alternatively, said animals may be immunised with peptides derived from the extracellular domain of CCR2, preferably peptides representing the loop1 and loop2 of the extracellular domain. Hybridoma cells are then screened for a binding to CCR2-positive cells and specificity can be ensured by comparing this binding to the binding to CCR2-negative cells. Alternatively, the binding to said peptides derived from the extracellular domain of CCR2 is compared to control peptides.

A more detailed description of the production of CCR2-specific antibodies, in particular of antibodies DOC-2 and DOC-2 is provided in Example 9 below.

Preferably, the antibodies of the invention contain or are coupled to domains, which enhance their depleting effect of the antibodies. Such domains are known to the person skilled in the art. An example for that is an Fc domain inducing ADCC or complement activation.

"Small molecular substances binding to CCR2", in accordance with the present invention, include without being limiting INCB3344 (Shin *et al.* 2009), 2-thioimidazoles (Doyon *et al.* 2008) as well as the compounds reviewed by Paul J. Higgins, C. Eric Schwartz und Jean-Marie Nicolas in Chemokine Biology - Basic Research and Clinical Application; Progress in Inflammation Research, 2007, Part 2, 115-123 entitled "Small molecule CCR2 antagonists" such as e.g. RS-504393, Benzimidazoles, SB-380732, AZD-6942, 3-Aminopyrrolidines or INCB-003284.

The agent may further be a "chemokine or modified chemokine binding to CCR2". Such chemokines are well known in the art and include, without being limiting CCL2, CCL8, CCL16, CCL7 and CCL13.

CCL2 refers to "chemokine (C-C motif) ligand 2", also referred to in the art as monocyte chemotactic protein-1 (MCP-1) and small inducible cytokine A2. Human CCL2 has been assigned Gene ID: 6347.

CCL8 refers to "chemokine (C-C motif) ligand 8", and is also referred to in the art as monocyte chemotactic protein-2 (MCP-2). Human CCL8 has been assigned Gene ID: 6355.

CCL16 refers to "chemokine (C-C motif) ligand 16", also referred to in the art as Liver-expressed chemokine (LEC) and Monotactin-1 (MTN-1). Human CCL2 has been assigned Gene ID: 6360.

CCL7 refers to "chemokine (C-C motif) ligand 7" and was previously called monocyte-specific chemokine 3 (MCP3). Human CCL13 has been assigned Gene ID: 6354.

CCL13 refers to "chemokine (C-C motif) ligand 13. Human CCL13 has been assigned Gene ID: 6357.

It is preferred that the agent is an agent specifically binding to CCR2. In accordance with the present invention, an agent is considered to bind specifically to CCR2 (also referred to herein as interacting specifically with CCR2) when the respective agent does not or essentially does not cross-react with a molecule that is not the C-C chemokine receptor type 2, in particular similar proteins such as e.g. CCR5, CCR1 or CCR3. Cross-reactivity of a panel of agents under investigation may be tested, for example, by assessing binding of said panel of agents under conventional conditions to the C-C chemokine receptor type 2 as well as to a number of more or less (structurally and/or functionally) closely related molecules. Only those molecules that bind to the C-C chemokine receptor type 2 but do not or do not essentially bind to any of the other molecules are considered specific for the CCR2. Corresponding methods are described e.g. for antibodies in Harlow and Lane, Antibodies: A Laboratory Manual, Cold Spring Harbor Laboratory Press, 1988 or in Harlow and Lane, Using Antibodies: A Laboratory Manual, Cold Spring Harbor Laboratory Press, 1999). The term "an agent that essentially does not cross-react with a molecule that is not the C-C chemokine receptor type 2", as used herein, refers to an agent that binds to the CCR2 with at least 5-times higher affinity than to a molecule of similar structure, more preferably at least 10-times higher affinity, such as e.g. at least 50-times higher affinity, more preferably at least 100-times higher affinity, such as e.g. at least 500-times higher affinity. Even more preferably, it binds with at least 1.000-times higher affinity to the CCR2 than to a molecule of similar structure, such as e.g. at least 5.000-times higher affinity and most preferably at least 10.000-times higher affinity. It will be appreciated that cross-reactivity with orthologous CCR2 receptors from other species is not excluded in accordance with the present invention and can even be advantageous in order to allow an application of the agent in several species. Such cross-reactivity is not uncommon and it is known to the person skilled in the art how to determine the cross-reactivity.

Preferably, the agent capable of specifically binding to CCR2 and inducing the depletion of CCR2-positive monocytes in a subject is an antibody, such as e.g. the antibodies shown in the appended examples. More preferably, the antibody is an antibody obtainable as characterised in Example 9.

In accordance with the present invention, the subject is a vertebrate, preferably a mammal. A mammal may be selected, for example, from the group consisting of a rodent, a pig, a dog, a cat or a primate.

Non-limiting examples of "rodents" are mice, rats, squirrels, chipmunks, gophers, porcupines, beavers, hamsters, gerbils, guinea pigs, degus, chinchillas, prairie dogs, and groundhogs. Preferably, the rodents are mice, rats or rabbits.

Non-limiting examples of "dogs" include members of the subspecies *canis lupus familiaris* as well as wolves, foxes, jackals, and coyotes. Preferably, the dogs are from the subspecies *canis lupus familiaris* and in particular are selected from beagles or dobermans.

Non-limiting examples of "cats" include members of the two subfamilies: the pantherinae, including lions, tigers, jaguars and leopards and the felinae, including cougars, cheetahs, servals, lynxes, caracals, ocelots and domestic cats.

The term "primates", as used herein, refers to all monkeys including for example cercopithecoid (old world monkey) or platyrrhine (new world monkey) as well as lemurs, tarsiers, apes and marmosets (Callithrix jacchus) as well as humans. Preferably, the primates are selected from the group consisting of marmosets as well as guenons, macaques, capuchins, squirrel monkeys and humans.

It is particularly preferred that the subject is a primate. Most preferred, the subject is a human subject.

Inflammatory diseases of the gastrointestinal system are well known in the art (Vermeire *et al.* 2012) and include, without being limiting, the diseases specifically discussed herein below.

In accordance with the present invention, a novel sub-population of monocyte-derived macrophages expressing Ly6C but only intermediate levels of CX₃CR1 was identified in a murine model of IBD induced by dextran sodium sulfate (DSS).

As is shown in the appended examples, differential fates of blood monocytes were investigated herein in the healthy and inflamed colon of mice. It was shown that Ly6C^{hi} monocytes differentiate in healthy tissue into resident CX₃CR1^{hi} MΦs acquiring a characteristic non-inflammatory gene expression profile. In contrast to their homeostatic differentiation fate, upon CCR2-dependent recruitment into acutely inflamed colonic tissue, Ly6C^{hi} monocytes activate TLR/NOD2 recognition pathways to become responsive to bacterial products and give rise to IL-6 and IL-23 secreting pro-inflammatory effector cells that critically promote colitis. With time, however, monocytes differentiate further into CX₃CR1^{int}Ly6C^{lo} CD11c⁺MHCII^{hi} cells and acquire concomitantly with increased CCR7 expression the ability to migrate to draining lymph nodes (LNs), as well as the potential to stimulate naive T cells towards luminal gut antigens.

Recruitment of the Ly6C^{hi} monocytes to the site of inflammation requires their expression of the chemokine receptor CCR2. Using a mixed monocyte adoptive transfer approach composed of wt and CCR2-deficient Ly6C^{hi} monocytes it could be shown that CCR2 expression is absolutely required for their recruitment to the inflamed colon. This finding suggests an active role of the Ly6C^{hi} monocytes and their descendants in DSS induced colitis, that is confirmed by the finding that the selective ablation of Ly6C^{hi} monocytes and their immediate CX₃CR1^{int}Ly6C^{lo} progeny, while sparing resident MΦs and CD103⁺ DCs (data not shown), ameliorates colitis of DSS-treated animals as manifested in significantly reduced colonoscopic colitis scores, decreased body weight loss and reduced expression and secretion of pro-inflammatory cytokines. Importantly, a functionally similar population expressing CD14, CD33 and TREM1 has been identified within inflamed intestinal tissues of both CD and UC patients.

Thus, the present findings not only enable for a high-resolution characterization of monocyte-derived cells with respect to their origin, trafficking requirements, pathways of differentiation and comprehensive gene expression signatures, but also improve the current understanding of the pathogenesis of human IBD and paves the road for novel therapeutic interventions based on strategies aiming at blocking monocyte influx.

The present invention further relates to a method of treating and/or preventing an inflammatory disease of the gastrointestinal system comprising administering a pharmaceutically effective amount of an agent capable of binding to CCR2 and inducing the depletion of CCR2-positive monocytes in a subject to a subject in need thereof.

The definitions and preferred embodiment provided herein with regard to the agent of the invention apply *mutatis mutandis* also to this method of the invention.

In another preferred embodiment of the agent of the present invention, the agent has admixed thereto or associated in a separate container one or more agents selected from the group consisting of antibiotic compounds, 5-aminosalicylates (5-ASA), steroids, immunomodulators, in particular TNF-alpha blockers, azathioprine and calcineurin inhibitors. Furthermore, in a further preferred embodiment of the method of the invention, the method further comprises the administration of one or more agents selected from the group consisting of antibiotic compounds, 5-aminosalicylates (5-ASA), steroids, immunomodulators, in particular TNF-alpha blockers, azathioprine and calcineurin inhibitors.

Antibiotic compounds, in accordance with the present invention, are antimicrobial compounds, i.e. compounds capable of killing microbes or inhibiting their growth. Preferably, the antibiotic compounds are antibacterial compounds. Non-limiting examples of antibiotics suitable for use in accordance with this preferred embodiment include metronidazole, ciprofloxacin, a combination of metronidazole and ciprofloxacin, rifaximin, clarithromycin, rifabutin, clofazamine, azithromycin, amoxicillin, tetracycline, and vancomycin (reviewed in Rahimi *et al.* 2007). Combination of the agent of the present invention with antibiotic compounds provides the advantage of increasing positive therapeutic effects of different treatment approaches while minimizing the unwanted side effects by lowering the dose or treatment duration of each individual treatment approach.

5-aminosalicylates (5-ASA) include, for example, sulfasalazine and mesalamine (Riley *et al.* 1988). Combination of the agent of the present invention with 5-aminosalicylates also provides the advantage of of increasing positive therapeutic effects of different treatment approaches while minimizing the unwanted side effects by lowering the dose or treatment duration of each individual treatment approach.

Glucocorticoids are a class of steroid hormones that bind to the glucocorticoid receptor. Glucocorticoids include, for example, budesonide and prednisone (Turner *et al.* 2007; Marshall and Irvine, 1997). Combination of the agent of the present invention with glucocorticoids provides the advantage of increasing positive therapeutic effects of different treatment approaches while minimizing the unwanted side effects by lowering the dose or treatment duration of each individual treatment approach.

Immunomodulators are compounds capable of up-or down-regulating the immune system and include, without being limiting, azathioprine, 6-mercaptopurine and methotrexate (George *et al.* 1996; Pearson *et al.* 1995). A further, preferred, class of immunomodulators are TNF-alpha blockers, which are inhibitors of TNF-alpha signaling and include, for example, infliximab, natalizumab, adalimuma and certolizumab (Ford *et al.* 2011). Combination of the agent of the present invention with immunomodulators provides the advantage of of increasing positive therapeutic effects of different treatment approaches while minimizing the unwanted side effects by lowering the dose or treatment duration of each individual treatment approach.

Calcineurin inhibitors are compounds capable of inhibiting the function of the calcium-dependent serine-threonine phosphatase calcineurin, such as for example cyclosporine A (Lichtiger *et al.* 1994). Combination of the agent of the present invention with calcineurin inhibitors provides the advantage of of increasing positive therapeutic effects of different treatment approaches while minimizing the unwanted side effects by lowering the dose or treatment duration of each individual treatment approach.

The present invention further relates to a pharmaceutical composition comprising the agent of the present invention, and optionally a pharmaceutically acceptable carrier.

In accordance with the present invention, the term "pharmaceutical composition" relates to a composition for administration to a patient, preferably a human patient. The pharmaceutical composition of the invention comprises the compounds or combination of compounds recited above. The pharmaceutical composition of the present invention may, optionally and additionally, comprise a pharmaceutically acceptable carrier. By "pharmaceutically acceptable carrier" is meant a non-toxic solid, semisolid or liquid filler, diluent, excipient, encapsulating material or formulation auxiliary of any type. Examples of suitable pharmaceutical carriers are well known in the art and include sodium chloride solutions, phosphate buffered sodium chloride solutions, water, emulsions, such as oil/water emulsions, various types of wetting agents, sterile solutions, organic solvents etc. Preferably the carrier is a parenteral carrier, more preferably a solution that is isotonic with the blood of the recipient. The term "parenteral" as used herein refers to modes of administration, which include intravenous, intramuscular, intraperitoneal and subcutaneous injection and infusion as well as local administration in the bowel. These modes of administration, together intramuscular, intradermal, intranasal or intrabronchial administration, are preferred modes of administration in accordance with the present invention. The carrier optionally contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or further immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

Compositions comprising such carriers can be formulated by well known conventional methods. Generally, the formulations are prepared by contacting the components of the pharmaceutical composition uniformly and intimately with liquid carriers or finely divided solid carriers or both. Then, if necessary, the product is shaped into the desired formulation.

These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgment of the ordinary clinician or physician. The pharmaceutical composition may be for administration once or for a regular administration over a prolonged period of time. Generally, the administration of the pharmaceutical composition should be in the range of for example 0.1µg/kg of body weight to 200 mg/kg of body weight for a single dose of the active compound of the present invention. However, a more preferred dosage of the active compound might be in the range of 10µg /kg to 50 mg/kg of body weight, even more preferably 0.5mg/kg to 20mg/kg of body weight and even more preferably 1 mg/kg to 10mg/kg of body weight for a single dose. Adjustment of these dosages in case of combination of the agent of the present invention with further pharmaceutically active compounds, such as those described herein above, is within the skills and judgment of the ordinary clinician or physician.

The components of the pharmaceutical composition to be used for therapeutic administration must be sterile. Sterility is readily accomplished by filtration through sterile filtration membranes (e.g., 0.2 micron membranes).

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic water-for-injection. Preservatives and other additives may also be present such as, for example, antimicrobials, anti oxidants, chelating agents, and inert gases and the like. The pharmaceutical composition may comprise further agents depending on the intended use of the pharmaceutical composition.

The pharmaceutical composition may be particularly useful for the treatment of inflammatory disease of the gastrointestinal system, as disclosed below.

In another preferred embodiment of the agent or the method of the invention, the inflammatory disease of the gastrointestinal system is selected from the group consisting of Crohn's disease, ulcerative colitis, collagenous colitis, lymphocytic colitis, diversion colitis, Behçet's disease, celiac disease, Whipple's disease, vasculitis affecting the intestine and indeterminate colitis.

Crohn's disease is also known as granulomatous colitis and regional enteritis and is an inflammatory disease that may affect any part of the gastrointestinal tract from mouth to anus. Symptoms of Crohn's disease can vary significantly among afflicted individuals. The main gastrointestinal symptoms are abdominal pain, diarrhea (which may be visibly bloody), vomiting, or weight loss. Crohn's disease can also cause complications outside of the gastrointestinal tract such as skin rashes, arthritis, and inflammation of the eye. The precise cause of Crohn's disease is not known. The disease occurs when the immune system attacks the gastrointestinal tract. This autoimmune activity produces inflammation in the gastrointestinal tract, and therefore Crohn's disease is classified as an inflammatory bowel disease (Vermeire *et al.* 2012).

Ulcerative colitis is a disease of the intestine, specifically the large intestine or colon, that includes characteristic ulcers, or open sores, in the colon. The main symptom of active disease is usually diarrhea mixed with blood, of gradual onset. Ulcerative colitis is, however, a systemic disease that affects many parts of the body outside the intestine (Garud and Peppercorn 2009).

Collagenous and lymphocytic colitis are well-described conditions causing chronic watery diarrhoea. (Chande *et al.* 2006; Temmerman and Baert 2009).

Diversion colitis is an inflammatory process that occurs in segments of the colorectum that are diverted from the fecal stream by surgery (Szczepkowski *et al.* 2008).

Behçet's disease is characterized by recurrent oral aphthae and any of several systemic manifestations including genital aphthae, ocular disease, skin lesions, gastrointestinal involvement, neurologic disease, vascular disease, or arthritis. Behçet's may have been described by Hippocrates but was brought to the attention of the modern medical community by Hulusi Behçet in 1937 (Feigenbaum 1956).

Celiac disease (also called gluten-sensitive enteropathy and nontropical sprue) can be defined as a small bowel disorder characterized by mucosal inflammation, villous atrophy, and crypt hyperplasia, which occur upon exposure to dietary gluten and which demonstrate improvement after withdrawal of gluten from the diet (Rostom *et al.* 2004).

Whipple's disease is a rare bacterial infection that most often affects our gastrointestinal system.

Whipple's disease interferes with normal digestion, impairing the breakdown of foods, such as fats and carbohydrates, and hampering the body's ability to absorb nutrients (Vangoitsenhoven *et al.* 2010).

Vasculitis involving the gastrointestinal tract is usually part of a systemic process although the signs and symptoms may initially be limited (Watts and Scott 2009).

Indeterminate colitis refers to diseases showing symptoms of colitis where no unambiguous or specific diagnosis is possible.

All of the diseases described herein are well known to the skilled person and are defined in accordance with the prior art and the common general knowledge of the skilled person.

The figures show:

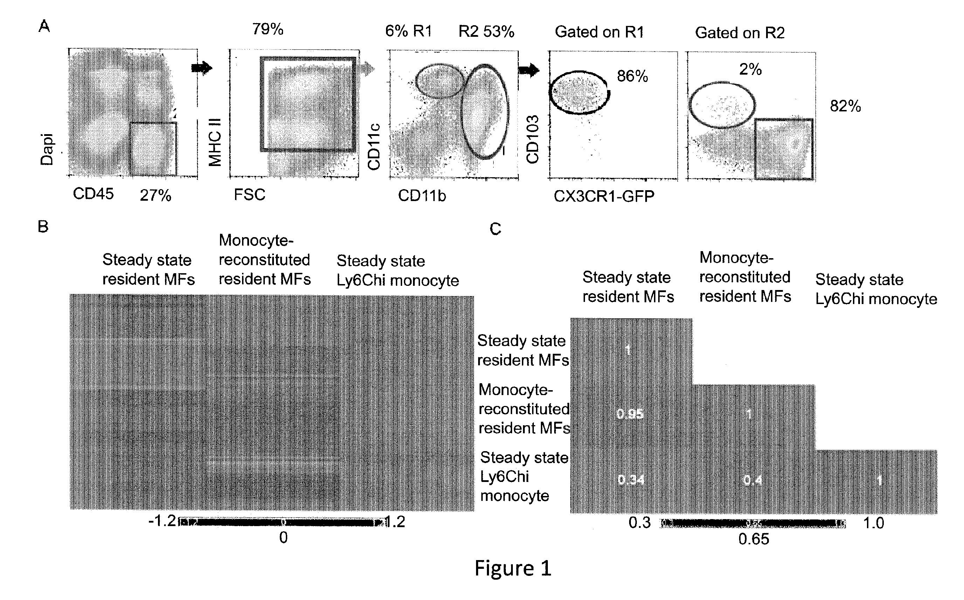

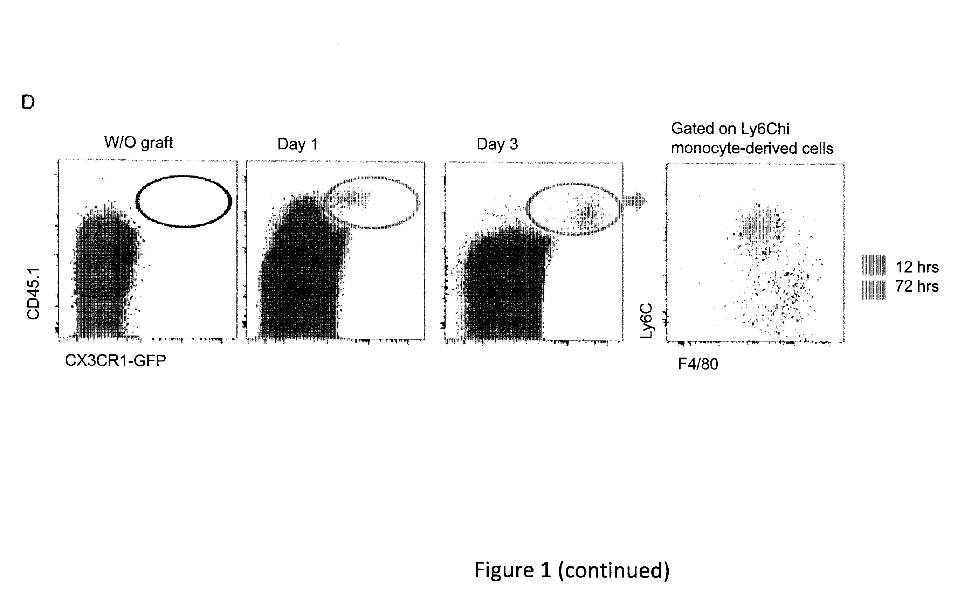

**Figure 1****. Steady state colonic lamina propria predominantly harboring Ly6C^{hi} monocyte-derived CX₃CR1/GFP^{hi} M**Φ**s**
   **(A)** Flow cytometry analysis of steady state colonic lamina propria of CX₃CR1^{+/gfp} mice indicating definitions and gating strategy. Note that the CX₃CR1/GFP ^{hi} MΦs constitute the most prominent mononuclear phagocyte subset in steady state.
   **(B)** Based on the microarray data, a total of 4412 differentially expressed genes were chosen, with at least 2 fold change between any of the three investigated cell populations: Steady state CX₃CR1/GFP^{hi} MΦs (pooled isolate of 10 mice), Ly6C^{hi} monocyte-graft-derived CX₃CR1/GFP^{hi} MΦs (pooled isolate of 14 engrafted [CD11c-DTR>wt] BM chimeras, 14 days after monocyte transfer), and Ly6C^{hi} monocytes (isolated from 6 spleens). The log 2 intensities were standardized to have for each gene zero mean and unit standard deviation. Hierarchical clustering of the standardized values was performed using the Pearson dissimilarity measure. The expression profile is accompanied by a bar indicating the standardized log 2 intensities.
   **(C)** The correlation matrix of pair-wise correlations (Pearson coefficient) describing the similarity between the different investigated cell populations is shown for the genes that are shown in Fig 1b. Correlations are grey coded according to the shown bar. Note the high similarity between CX₃CR1^{hi} resident MΦs isolated from steady state colon and colon mononuclear phagocyte-depleted mice reconstituted with Ly6C^{hi} monocytes.
   **(D)** Flow cytometry analysis of recipient colons (CD45.2) 1 and 3 days after endoscopy-assisted submucosal injection of 2x10⁵ Ly6C^{hi} monocytes (CX₃CR1^{gfp}, CD45.1). Note differentiation of Ly6C^{hi} monocytes into CX₃CR1/GFP ^{h1}F4/80^{hi} resident MΦs.

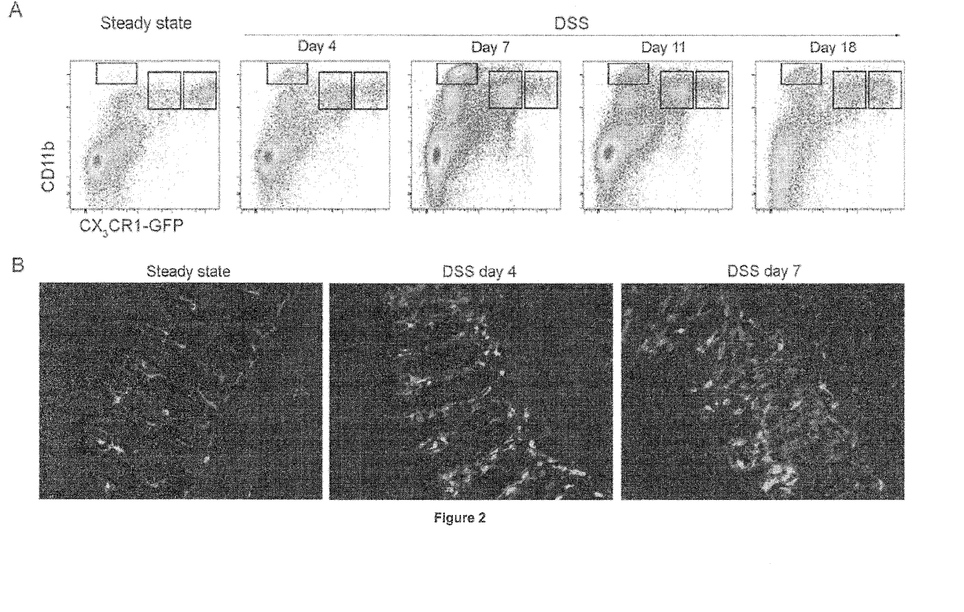

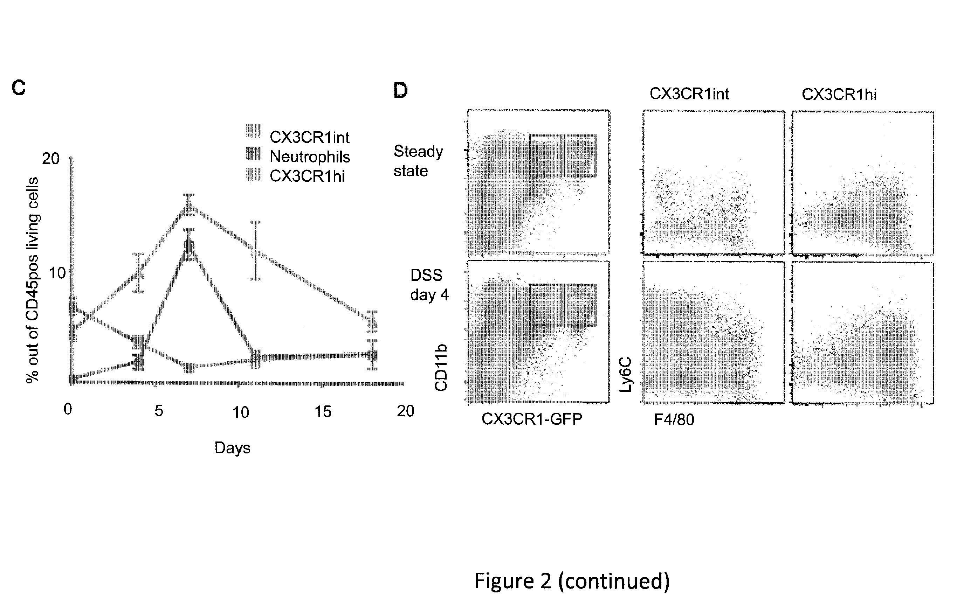

**Figure 2****. Colitic colon recruits CX₃CR1/GFP^{int}cells**
   **(A)** Representative flow cytometry plots gated on CD45⁺ living cells isolated from steady state colonic lamina propria of CX₃CR1^{+/gfp} mice and on days 4, 7, 11 and 18 after DSS challenge. Note progressive accumulation of CD11b^{hi}CX₃CR1/GFP^{int} cells and concomitant reduction of CX₃CR1/GFP ^{hi} resident MΦs.
   **(B)** Representative fluorescent microscopy images of CX₃CR1^{gfp/+} colons at steady state and day 4 and 7 after DSS challenge (green-CX₃CR1-gfp, blue-Hoechst, original magnifications 10x).
   **(C)** Dynamics of cellular infiltrates in colitic colon; CX₃CR1/GFP ^{int} cells (triangle), CX₃CR1/GFP ^{hi} resident-MFs (square) and CD11b^{hi}Gr1^{hi} neutrophils (circle), presented as percentages out of CD45⁺ living cells, mean (SEM) (n=4 for any time point).
   **(D)** Flow cytometry analysis of steady state and DSS day 4 colitis showing selective expression of Ly6C and F4/80 markers on CX₃CR1/GFP ^{int} and CX₃CR1/GFP ^{hi} mononuclear phagocytes, respectively. Results are representative of three or more independent experiments.

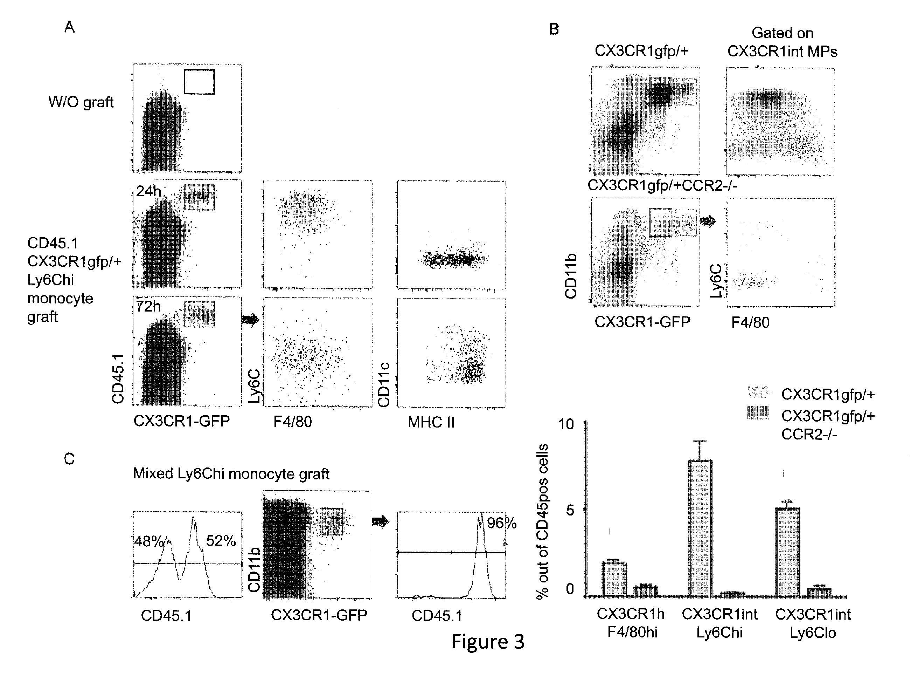

**Figure 3****. CX₃CR1/GFP ^{int} cells arise from Ly6C^{hi} monocytes under inflammatory conditions in a CCR2-dependent manner**
   **(A)** Flow cytometry analysis of DSS-challenged wt recipient mice (CD45.2) of Ly6C^{hi} monocytes (3x10⁶; CX₃CR1^{GFP/+}:CD45.1). Monocytes were engrafted on day 4 after onset of DSS treatment; analysis was performed 24 and 72 hrs post transplantation.
   **(B)** Flow cytometry analysis of colon of CX₃CR1^{+/gfp} and CX₃CR1^{+/gfp}CCR2^{-/-} mice for CD45⁺ living cells at day 5 after DSS onset. Graphical summary of percentage of indicated cell subset out of CD45⁺ cells, mean (SEM) (n=3)
   **(C)** Flow cytometry analysis of colon of CD45.2 wt recipient mice 72 hrs following intravenous adoptive transfer of Ly6C^{hi} monocyte graft composed of 1.5x10⁶ CX₃CR1^{+/gfp} (CD45.1) cells and 1.5x10⁶ CX₃CR1^{+/gfp}CCR2^{-/-} (CD45.2) cells at day 4 after onset of 2% DSS treatment. Results are representative of two independent experiments.

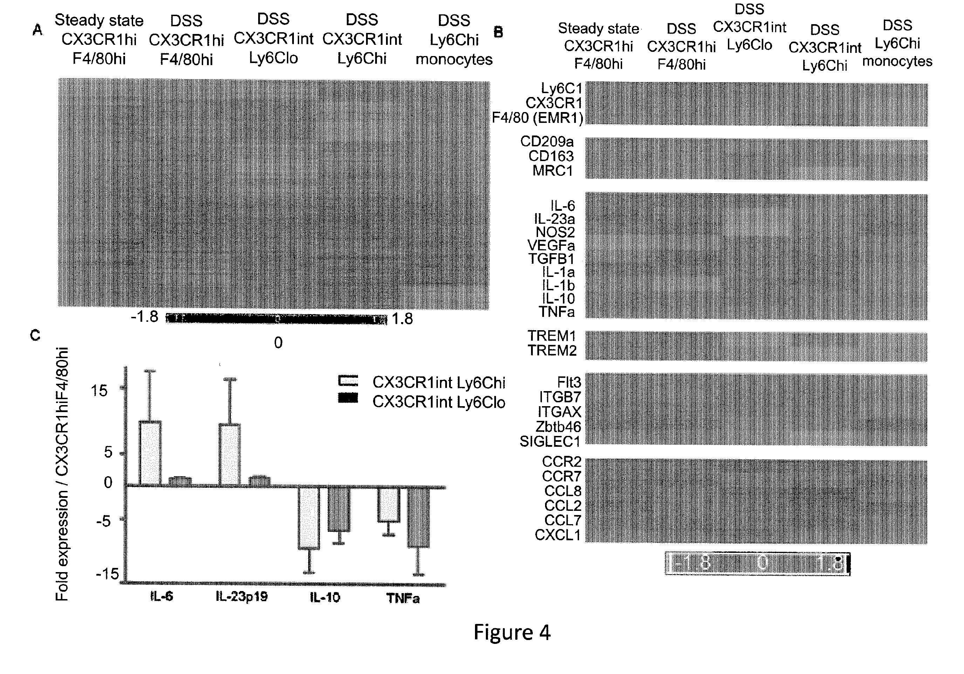

**Figure 4****. Molecular characterization of infiltrating CX₃CR1/GFP ^{int} and resident CX₃CR1/GFP ^{hi} F4/80^{hi} colonic mononuclear phagocytes**
   **(A)** Affymetrix microarray performed on mRNA of sorted CX₃CR1/GFP ^{hi}F4/80^{hi} resident MΦs [steady state (n=10), and DSS day 4 (n=12)], CX₃CR1/GFP ^{int}Ly6C^{hi} cells and CX₃CR1/GFP ^{int}Ly6C^{lo} [DSS day 4 (n=12)] and splenic Ly6C^{hi} monocytes [DSS day 4 (n=7)]. Genes which exhibited a fold change of at least 2 between any two of the investigated cell populations were chosen (5980 genes). The log 2 intensities were standardized to have for each gene zero mean and unit standard deviation. K-means partitioning clustering was performed using Pearson dissimilarity as a distance measure. The number of partition clusters was set to six. The expression profile is accompanied by a bar indicating the standardized log 2 intensities.
   **(B)** The heat map demonstrates expression changes for selected genes (derived from Fig 4A). The expression profile is accompanied by a bar indicating the standardized log 2 intensities.
   **(C)** Graphical summary of qRT-PCR analysis showing the mRNA level ratio of indicated cytokines between CX₃CR1^{int}Ly6C^{hi}, CX₃CR1^{int}Ly6C^{lo} mononuclear phagocytes and CX₃CR1^{hi}F4/80^{hi} resident MΦs sorted from colonic lamina propria of DSS-challenged mice (day 4). Results represent mean (SEM) of two independent experiments using pooled cells from 8-12 mice per experiment.

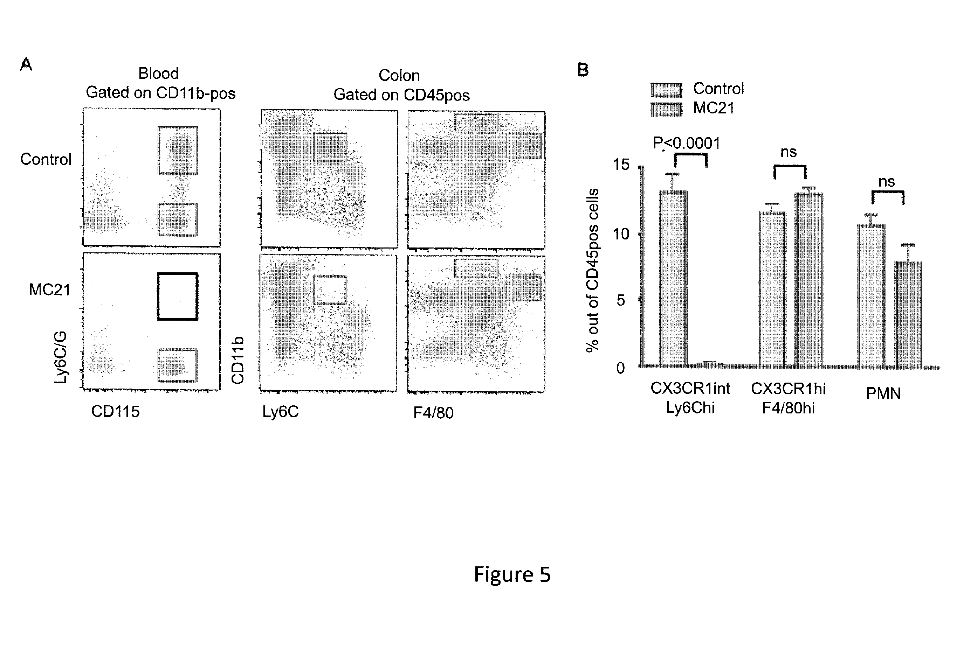

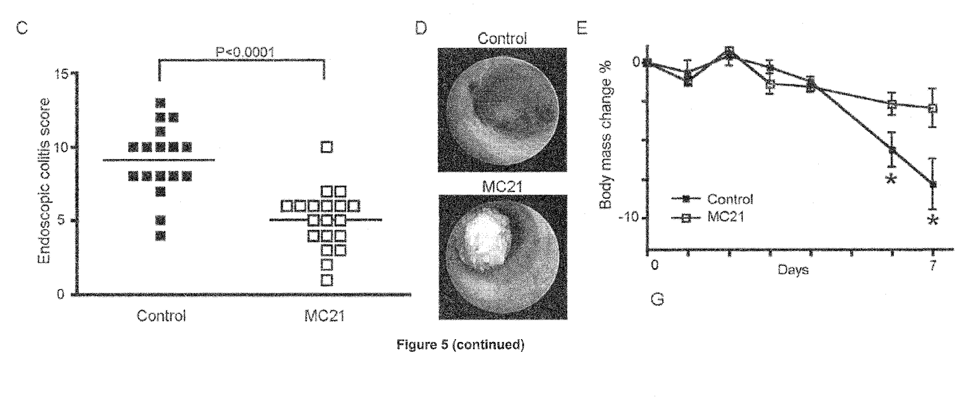

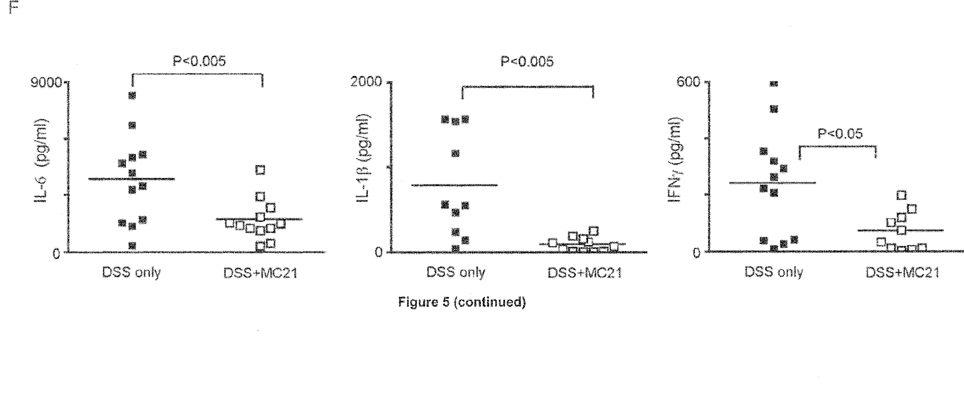

**Figure 5****. Ablation of CX₃CR1/GFP^{int} Ly6C^{hi} effector monocytes ameliorates DSS-induced colitis**
   (A) Flow cytometry analysis of blood (gated on CD11b⁺ cells) and colonic lamina propria (gated on CD45⁺ living cells) of mice subjected to DSS (day 7) and treated with MC21antibody or saline for 5 days. Note efficient selective ablation of blood Ly6C^{hi} monocytes, as well as of colonic lamina propria CD11b⁺Ly6C^{hi} effector monocytes.
   (B) Graphical summary of MC21 antibody treatment effect on colonic lamina propria MF subsets, as well as PMNs, mean (SEM) out of CD45⁺ living cells.
   (C) Graphical summary of endoscopic colitis grades assessed on day 7 after DSS initiation for mice left untreated or receiving concomitant MC21 antibody treatment (n=19 and n=17, respectively).
   (D) Representative colonoscopy images of indicated DSS-treated mice (day 7) with or without MC21 antibody treatment.
   (E) Graphical summary of body mass changes following DSS challenge of mice left untreated or receiving concomitant MC21 antibody treatment, * indicates p<0.05 at a specific time point.
   (F) Graphical summary of IL-6, IL-1b and IFNg levels determined by ELISA in colon explant culture supernatants extracts from colons of mice subjected to 7 day DSS treatment with or without MC21 antibody supplement. Results in this figure are representative of two or more independent experiments.

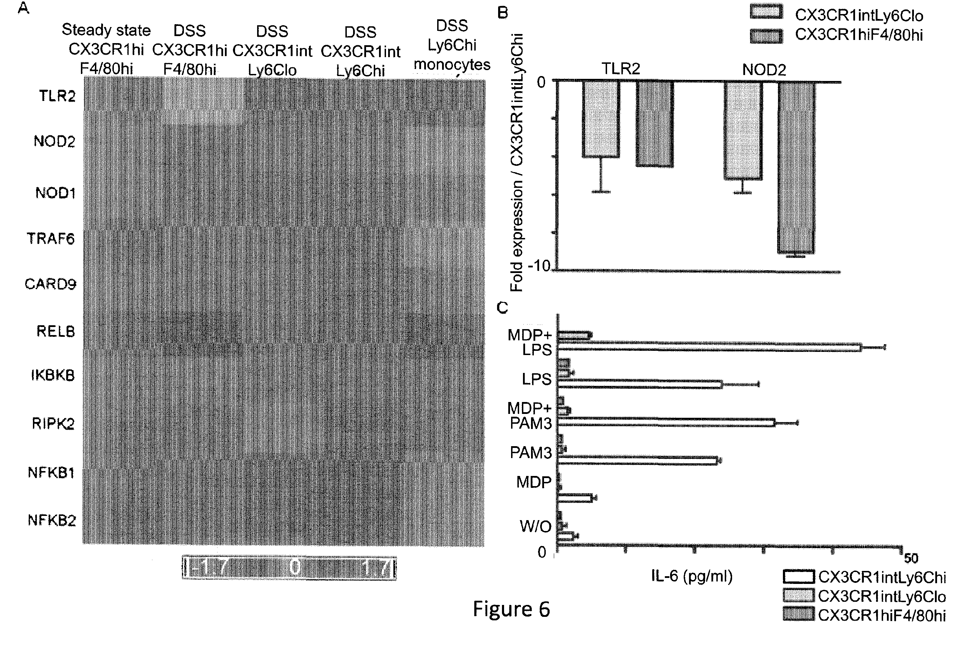

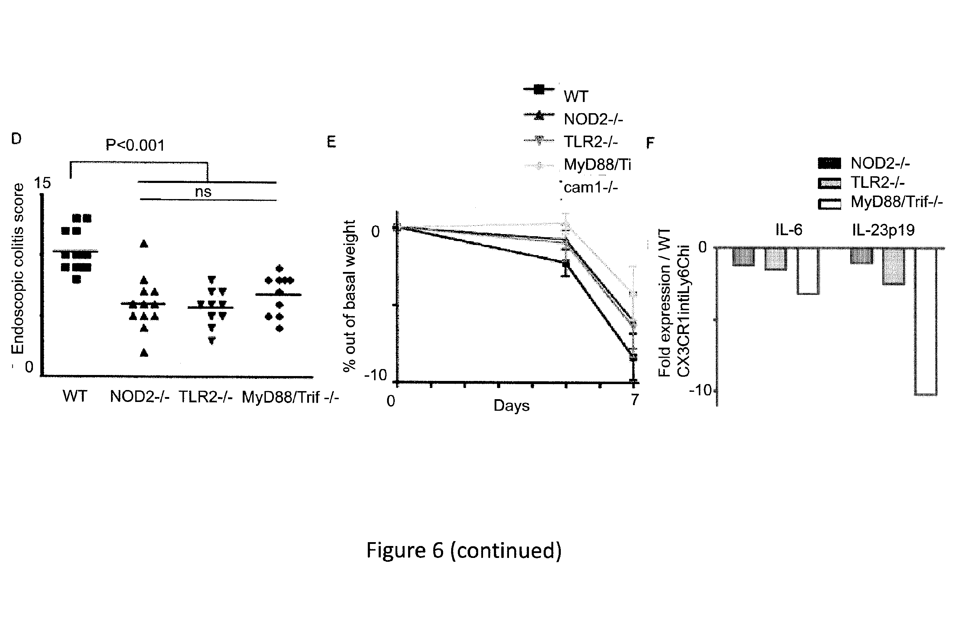

**Figure 6****. TLR2 / NOD2 ligands trigger pro-inflammatory activity of CX₃CR1/GFP^{int} Ly6C^{hi} effector monocytes**
   **(A)** Heat map demonstrating expression changes for genes involved in TLR2 / NOD signaling (derived from Fig 4A). Expression profile is accompanied by a bar indicating the standardized log 2 intensities.
   **(B)** Graphical summary of qRT-PCR analysis showing fold mRNA expression of TLR2 and NOD2 in CX₃CR1/GFP ^{hi} F4/80^{hi} resident MΦs and CX₃CR1/GFP ^{int}Ly6C^{lo} cells over CX₃CR1/GFP ^{int}Ly6C^{hi} effector monocytes. Graph depicts means (SEM) of two independent experiments, each of which comprised pooled RNA from 8-12 mice.
   **(C)** ELISA results of IL-6 production by CX₃CR1/GFP ^{int}Ly6C^{hi} effector monocytes, CX₃CR1/GFP ^{int}Ly6C^{lo} cells and CX₃CR1/GFP ^{hi}F4/80^{hi} resident MΦs sorted from the colonic lamina propria at DSS day 7 and cultured for 16 hr with MDP (Enzo Life Science) (10 mg/ml), Pam3CSK4 (InvivoGen) (10 µg/ml), LPS (Sigma) (100 ng/ml) or combinations of the above. Results are representative of two independent experiments.
   **(D)** Graphical summary of endoscopic colitis grades assessed on day 7 after DSS initiation for [TLR2^{-/-} > wt], [NOD2^{-/-}> wt] and [Myd88^{-/-} Trif^{-/-} > wt] BM chimeras, as well as [wt > wt] controls (n=19 and n=17, respectively).
   **(E)** Graphical summary of body mass changes of DSS-treated [TLR2^{-/-} > wt], [NOD2^{-/-} > wt] and [Myd88^{- /-} Trif^{-/-} > wt] BM chimeras, as well as [wt > wt] controls (n=19 and n=17, respectively). Note that the observed differences in body mass change did not reach significance between the groups, though by their tendency support the notion of ameliorated colitis [TLR2^{-/-} > wt], [NOD2^{-/-} > wt] and [Myd88^{-/-} Trif^{-/-} > wt] BM chimeras.
   (F) Graphical summary of qRT-PCR analysis showing fold mRNA expression of IL6 and IL23 (p19) of CX₃CR1/GFP ^{int}Ly6C^{lo} cells isolated from DSS-challenged [TLR2^{-/-} > wt], [NOD2^{-/-} > wt] and [Myd88^{-/-} Trif^{-/-} > wt] BM chimeras, as well as [wt > wt] controls.

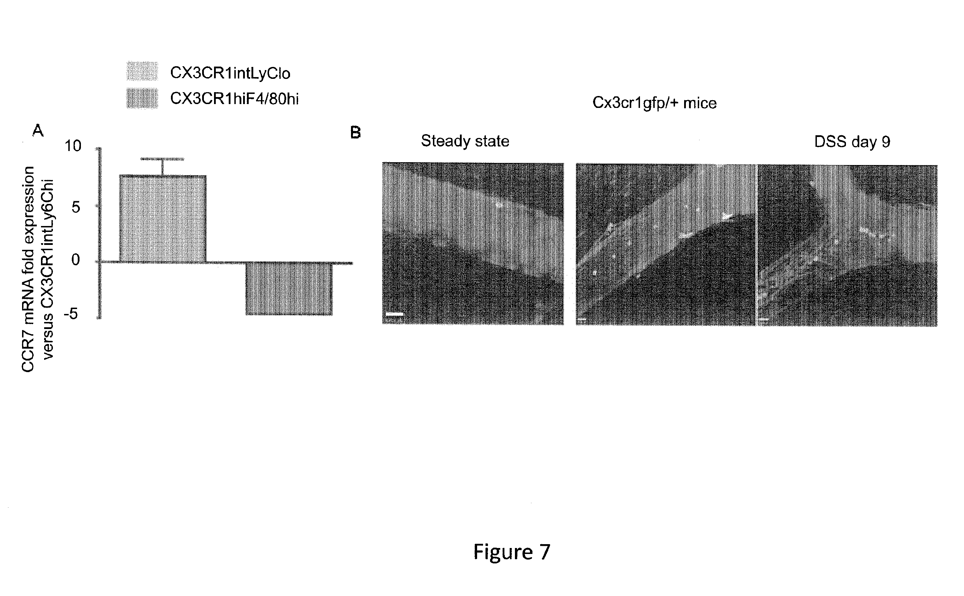

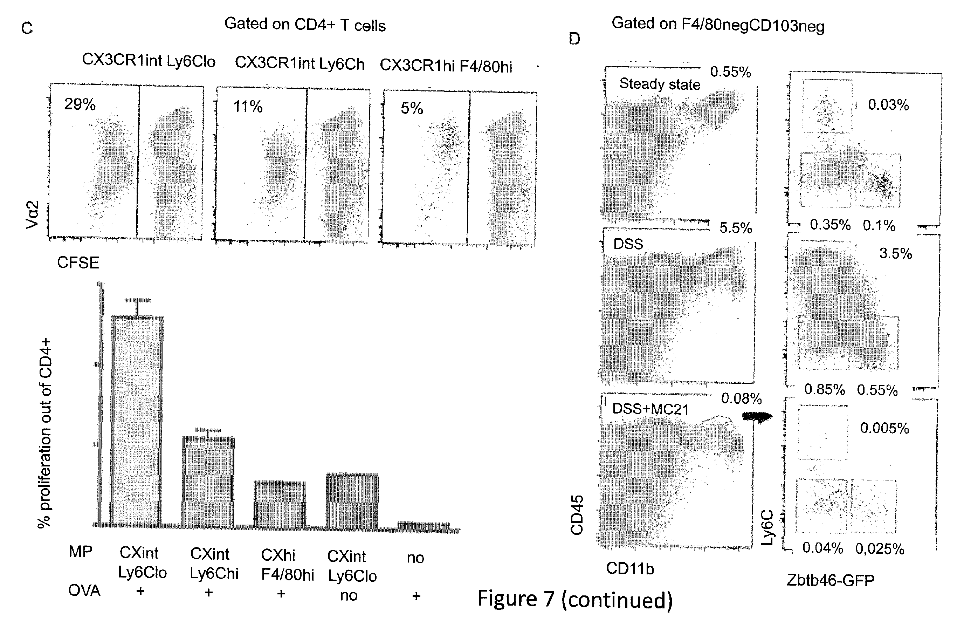

**Figure 7****. CX₃CR1/GFP ^{int}Ly6C^{lo} cells are migratory, acquire luminal antigens and can stimulate naive T cells.**
   **(A)** Graphical summary of qRT-PCR analysis of CCR7 expression by CX₃CR1/GFP^{int}Ly6C^{lo} and CX₃CR1/GFP ^{hi}F4/80^{hi} resident MΦs versus CX₃CR1/GFP^{int}Ly6C^{hi} cells isolated from colitic colon. Results are mean (SEM) of two independent experiments using pooled cells from 8-12 mice per experiment.
      Note that both CX₃CR1/GFP^{int}Ly6C^{hi} and Ly6C^{lo} cells express CCR7, albeit at distinct levels.
   **(B)** Representative pictures obtained by two photon microscopy imaging of lymphatic vessels draining the colon of CX₃CR1^{+/gfp} mice in steady state and at DSS day 9. Note accumulation of migratory CX₃CR1-GFP⁺ cells in the lymphatic vessels of colitic tissue.
   **(C)** Flow cytometry analysis of OVA-specific CFSE-labelled CD4⁺ T cells (OT II) 3.5 days following their co-culture with CX₃CR1/GFP^{int}Ly6C^{lo} cells, CX₃CR1/GFP^{int}Ly6C^{hi} effector monocytes or CX₃CR1^{hi}F4/80^{hi} resident MΦs (1:2 ratio) isolated from DSS day 7 colon of CX₃CR1^{GFP/+} mice gavaged with 100 mg OVA 1 day earlier. Graph summarizes percentages of proliferated / CFSE^{lo} CD4⁺ T cells out of total CD4⁺ T cells. Results represent mean (SEM) of three independent experiments using pooled cells from three mice per experiment for CX₃CR1/GFP^{int}Ly6C^{lo} and CX₃CR1/GFP^{int}Ly6C^{hi} cells, and a pool of seven mice for CX₃CR1/GFP^{hi}F4/80^{hi} cells.
   **(D)** Flow cytometry analysis of colonic lamina propria of [zbtb46^{gfp} > wt] BM chimeras subjected to DSS (day 7) treated with MC21 antibody or saline for 5 days. Note presence of zbtb46/GFP⁻ and zbtb46/GFP⁺ CX₃CR1/GFP ^{int}Ly6C^{lo} cells.

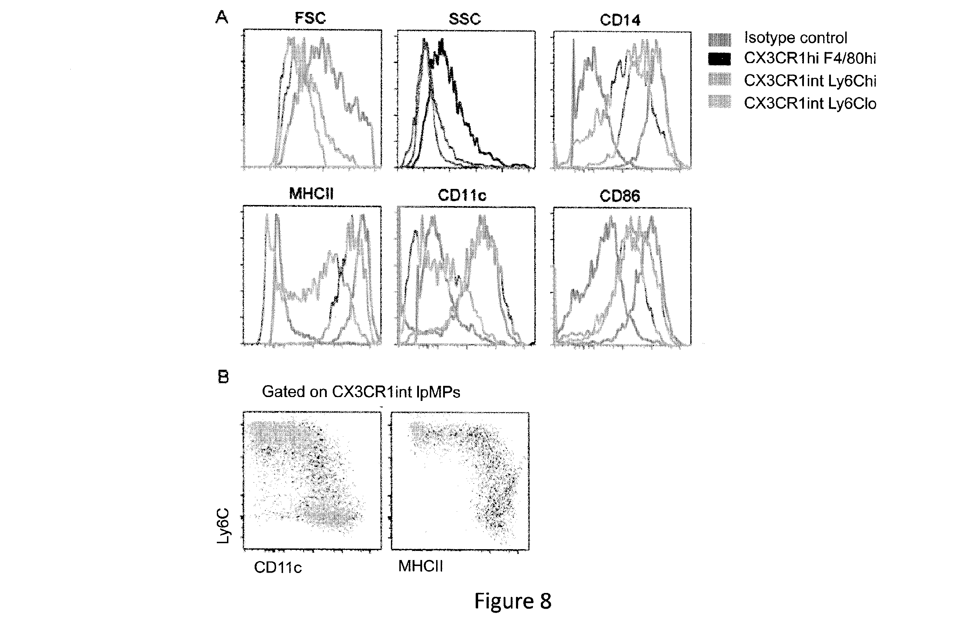

**Figure 8****. Phenotypic characterization of Ly6C^{hi} monocyte-derived mononuclear phagocyte subsets in the inflamed colon**
   **(A)** Flow cytometric analysis of colonic lamina propria cells of CX₃CR1^{GFP/+} mice subjected to DSS for 4 days focusing on monocyte-derived CX₃CR1/GFP^{int}Ly6C^{hi} cells, CX₃CR1/GFP^{int}Ly6C^{lo} cells and CX₃CR1/GFP^{hi}F4/80^{hi} MFs.
   **(B)** Flow cytometric analysis of CD11c and MHCII on CX₃CR1/GFP^{int} cells isolated from colon of CX₃CR1^{GFP/+} mice subjected to DSS for 4 days. Note the induction of CD11c and MHC II expression on CX₃CR1/GFP^{int}Ly6C^{lo} cells.

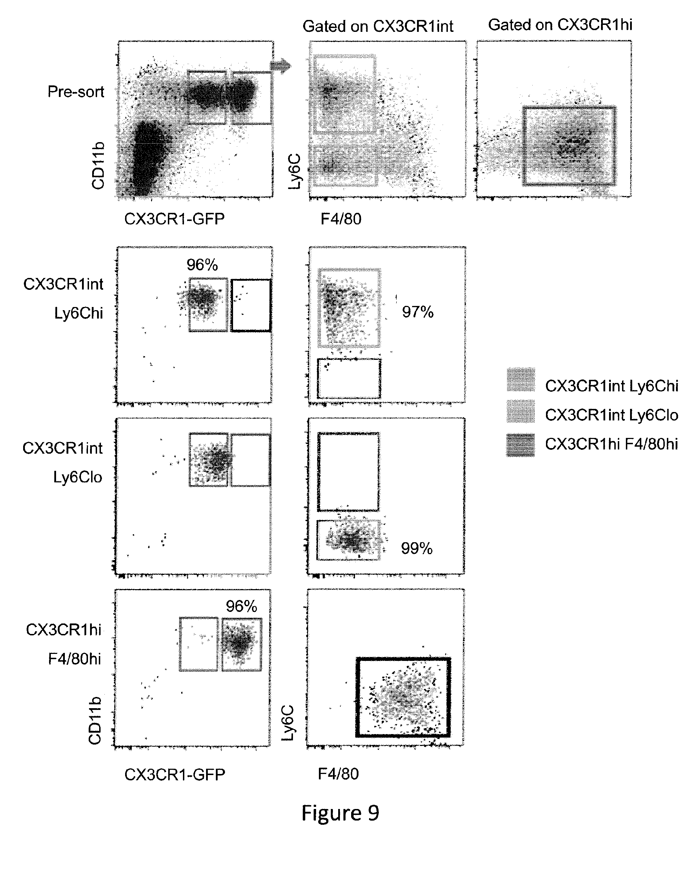

**Figure 9****. Flow cytometry sorting strategy used for isolation of monocyte-derived mononuclear phagocyte subsets from colon of CX₃CR1^{+/gfp} mice (DSS day 4)** Flow cytometry analysis indicating strategy used to identify and sort CX₃CR1/GFP ^{int}Ly6C^{hi} effector monocytes, CX₃CR1/GFP^{int} Ly6C^{lo} cells and CX₃CR1/GFP^{hi}F4/80^{hi} resident MFs. Results are representative of more than 5 independent experiments using pooled cells from 8-12 mice per experiment.

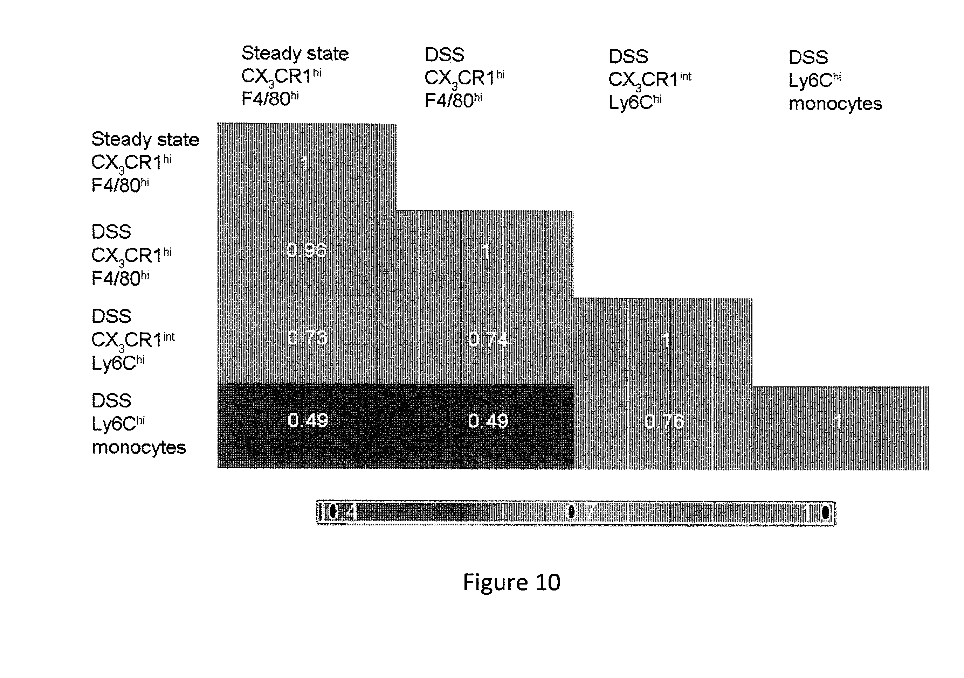

**Figure 10****. Pearson correlation matrix comparing gene expression profiles of CX₃CR1/GFP^{hi} resident MFs, CX₃CR1/GFP^{int}Ly6C^{hi} cells, CX₃CR1/GFP^{int}Ly6C^{lo} cells and Ly6C^{hi} splenic monocytes**
   Genes which exhibited a fold change of at least 2 between any two of the investigated five cell populations were chosen (5980 genes; a heat map of these genes is shown in Figure 4A in the manuscript). Correlation matrix of the pair-wise correlations (Pearson coefficient) describing the similarity between the different investigated cell populations is shown for the selected genes. Correlations are color-coded according to the shown bar.
**Figure 11****. Selective short-term cell ablation using anti-CCR2 antibody**
   Flow cytometry analysis for colon of CX3CR1^{+/gfp} mice subjected to DSS (day 7) and treated with MC21 for 5 days versus untreated controls.
**Figure 12****. The effect of MC21-induced ablation of CX₃CR1/GFP^{int} cells on colonic cytokines**
   Graphical summary of qRT-PCR analysis of IL-6, IL23p19, IL1b, TREM1, TGFb1 and IFNg expression in colons of mice subjected to DSS (day 7) with MC21 treatment versus controls and compared to a representative mouse in the MC21-treated group. Results are representative of two independent experiments.

The examples illustrate the invention:

### Example 1: Materials and methods

### Mice

C57BL/6 CD45.2 mice were purchased from Harlan. C57BL/6 CD45.1, OT II TCR transgenic mice harboring OVA-specific CD4+ T cells (Barnden et al., 1998), Cx3cr1^{gfp/+} mice (Jung et al., 2000), CD11c-DTR transgenic mice (B6.FVB-Tg [Itgax-DTR/GFP] 57Lan/J) (Jung et al., 2002), and CCR2^{-/-} Cx3cr1^{gfp/+} mice were bred at the Weizmann animal facility. MyD88/Trif DKO mice were kindly provided by Eran Elinav (Yale University). TLR2-deficient (Travassos et al., 2004) and NOD2-deficient (Kobayashi et al., 2005) mice were bred at the Institute Pasteur animal facility. Zbtb46gfp mice (Satpathy et al., 2012) served as BM donors. All mice were backcrossed against a C57BL/6 background. [DTR > WT] BM chimeras were generated as reported (Varol et al., 2007). After BM transfer, the recipients were allowed to rest for 8 weeks before use. All mice were maintained under specific pathogen-free conditions and handled according to protocols approved by the Weizmann Institute and the Institute Pasteur Animal Care Committee as per international guidelines.

### Cell Isolation, flow cytometry analysis and sorting of phagocyte subsets

Isolation of colonic lamina propria cells was performed by following a method established previously with slight modifications (Varol et al., 2009). In brief, extra-intestinal fat tissue and blood vessels were carefully removed and colons were then flushed of their luminal content with cold PBS, opened longitudinally and cut into 0.5-cm pieces. Epithelial cells and mucus were removed by 40 min incubation with HBSS (without Ca⁺⁺ and Mg⁺⁺) containing 5% FBS, 2mM EDTA, and 0.15 mg/ml (1mM) DTT (Sigma) at 37°C shaking at 250 rpm. Colons pieces were then digested in PBS^{+/+} containing 5% FBS, 1 mg/ml Collagenase VIII (Sigma) and 0.1 mg/ml DNase I (Roche) for 40 min at 37°C shaking at 250 rpm. The digested cell suspension was then washed with PBS and passed sequentially through 100- and 40-µm cell strainers. Antibodies used for colonic lamina propria staining included: CD45 (30-F11), CD45.2 (104), CD45.1 (A20), Ly6C (HK1.4), CD11c (N418), CD11b (M1/70), lAb (AF6-120.1), CD86 (GL-1) and CD14, all from BioLegend, CD103 (M290) BD Bioscience and F4/80 (Cl:A3-1) Serotec. For sorting strategy of lamina propria phagocyte subsets see Figure 9. BM cells were harvested from the femora and tibiae of CD45.1 Cx3cr1^{gfp/+} or CD45.2 CCR2^{-/-}Cx3cr1^{gfp/+} mice and enriched for mononuclear cells on a Ficoll density gradient. Ly6C^{hi} monocytes were then sorted by gating on cells positive for CD115, CD11b, CX₃CR1-GFP and Gr1 (Ly6C/Ly6G) and negative for CD117 (cKit). Spleens of Cx3cr1^{gfp/+} mice were mashed through 100- and 40-µm cell strainers, lyzed with ACK (0.15M NH₄Cl, 0.1M KHCO₃, 1mM EDTA in PBS) and enriched for monocytes by magnetic cell separation (MACS®) using biotinylated-conjugated CD115 antibody (AFS98) followed by streptavidin MACS beads (Miltenyi Biotec GmbH). Ly6C^{hi} monocytes were then sorted by gating on cells positive for CD115, CD11b, CX₃CR1-GFP and Gr1 (Ly6C/Ly6G). Other antibodies used herein: CD4 (GK1.5), Gr1 (RB6-8C5), CD115 (AFS98) BioLegend and Valpha 2 TCR (B20.1) Invitrogen. Cells were analyzed with LSRFortessa flow cytometer (BD) or sorted using a FACSAria machine (BD). Flow cytometry analysis was done with the FlowJo software.

### DSS-Induced Colitis Model and Murine Colonoscopy

Mice received one cycle (7 days) of dextran sulfate sodium salt (DSS) (MP Biomedicals, C-160110) treatment 2% in drinking water. To monitor and score colitis severity, we used a high-resolution murine video endoscopic system, consisting of a miniature probe (1.9 mm outer diameter), a xenon light source, a triple chip HD camera, and an air pump ("Coloview," Karl Storz) to achieve regulated inflation of the mouse colon. Digitally recorded video files were processed with Windows Movie Maker software (Microsoft). Endoscopic quantification of colitis was graded as previously described (Becker et al., 2006; Becker et al., 2005).

### Histology and Immunohistochemistry

Tissues were fixed in 2% paraformaldehyde overnight at 4°C and next impregnated in 30% sucrose (in PBS^{-/-}) for 48hrs. Sequentially, the colonic tissues were rapidly frozen in O.C.T. (Tissue-Tek) in isopentane cooled with liquid nitrogen, and then cut with cryostat to 12mm thick sections. Slides were observed with a Zeiss Axioscope II fluorescent microscope and image acquisition was conducted with simple PCI software.

### Microarray analysis

Total RNA was extracted from freshly isolated, flow cytometry-sorted cells, from CX₃CR1^{gfp/+} mice. Total RNA was extracted with the miRNeasy Mini Kit (Qiagen). RNA purity was assessed with ND-1000 Nanodrop (Peqlab) and BioAnalyzer 2100 (Agilent). The cDNA was prepared, labeled and hybridized to Affymetrix GeneChip, mouse gene 1.0 ST according to standard manufacturer protocols. Hybridized chips were stained and washed and were scanned using the Affymetrix GeneChip 3000 7G plus scanner. Affymetrix GeneChip Operating Software (GCOS v1.4, http://www.affymetrix.com) was used for the initial analysis of the microarray data to convert the image files to cell intensity files (CEL). Transcriptome analysis (processing of raw data, clustering, and creating correlation matrices) was carried out using Partek Genomic Suite 6.5 (Inc. St. Charles, MO; www.partek.com). The raw probe intensities were adjusted based on the number of G and C bases in the probe sequence, before any probe correction. Preprocessing was preformed using the Robust Microarray Averaging algorithm (Irizarry et al., 2003). Genes expressed below background levels (log 2 intensity of 5.5) in all examined conditions were removed from further analyses.

### Real time quantitative RT-PCR

Total RNA was extracted from murine colons with PerfectPure RNA Tissue Kit (5 PRIME), and from sorted cells with miRNeasy Mini Kit (Qiagen). RNA was reverse transcribed with a mixture of random primers and oligo-dT with a High-Capacity cDNA Reverse Transcription Kit (Applied Biosystems). PCRs of TBP, IL-6, IL-23p19, IL-10, TNFα, TLR-2, NOD-2, CCR7, E-cadherin, IL-1β, TREM-1, TGFβ1 and IFNγ were performed with SYBR Green PCR Master Mix kit (Applied Biosystems). Quantification of the PCR signals of each sample was performed by comparing the cycle threshold values (Ct), in duplicate, of the gene of interest with the Ct values of the TBP housekeeping gene.

### ELISA

The colons of DSS-treated mice were flushed with RPMI and opened along a longitudinal axis. Thereafter, 3 mm² punch biopsies were obtained from the distal colon and incubated for 24hrs in RPMI supplemented with 10% FCS and antibiotics (one punch biopsy per 100µl medium). Supernatants were collected and kept in -20°C until assessed. The production of the cytokines IL-6, IL-1β and IFNγ was assessed using the DuoSet ELISA kit (R&D Systems).

### Two-photon microscopy

To visualize cells in the intestinal lymphatics a protocol described before (Schulz *et al.* 2009) was modified. To label both lymphatic and blood vessels, 200 µl Evans blue (0.2%; Sigma) was injected i.v., 30 min before sacrifice. This dye leaks from blood vessels into the extracellular fluid and gathers in lymphatics where it can be visualized based on its red fluorescence. To distinguish blood vessels, the mice were further i.v. injected with high-MW dextran (MW 500,000; Sigma) labeled with FITC 2 min before sacrifice. Mice were then anesthetized with Ketamine/Xylazine 100/20 mg/kg. Laparotomy was performed, and the intestinal lymphatics were ligated with a suture close to the MLN. Mice were sacrificed and a 3-4 cm-long intestinal segment corresponding to the cecum and the proximal colon with its intact vasculature and the MLNs was dissected, glued into a cell culture dish, and submerged in cold PBS. The collected intestinal lymphatics were imaged using a 2-photon microscope (Ultima Multiphoton; Prairie Technologies) incorporating a pulsed laser (Mai Tai DeepSee Ti-sapphire; Newport Corp.) tuned to 930 nm to simultaneously excite Evans blue, FITC and GFP. A water-immersed 20x (NA 0.95) objective (Olympus) was used. Three-dimensional reconstruction of the lymphatics was performed with Improvision Volocity software.

### T cell proliferation assays

CD4⁺ Ova-specific T cells were isolated from spleen and mesenteric lymph node of TCR transgenic OT-II mice using magnetic cell separation technique (MACS®) with anti CD4 magnetic beads (Miltenyi Biotec GmbH). OT-II Cells were labeled with CFSE (C-1157, Invitrogen) and co-cultured with mononuclear phagocyte subsets sorted from the colonic lamina propria of mice subjected to DSS induced colitis and immunization with 100 mg OVA (Sigma Aldrich) by gavage the day before Ratio of T cell/APC was 2:1. Analysis of T cell proliferation was performed by flow cytometric analysis of CFSE dilution 3.5 days after co-culturing.

### Endoscopy guided colonic transplantation

Ly6C^{hi} monocytes were sorted to high purity (>95%) as described before and injected into the colonic lamina propria submucosal layer utilizing a previously described protocol established (Zigmond et al., 2011), which is based on the adaptation of the murine colonoscopy system. In brief, Ly6C^{hi} monocytes were microinjected (50µl volume) directly into the colonic lamina propria using the mouse colonoscopy system together with custom-made 30 gauge stainless steel flexible hypodermic needles with a 45° bevel (Cadence Inc', USA) that are passed through a Luer lock screwed on the working channel of the scope.

### Statistical Analysis

Data were analyzed either by ANOVA followed by Bonferroni's Multiple Comparison test or by unpaired, two-tailed t-test using GraphPad Prism 4 (San Diego, CA). Data are presented as mean ± SEM; values of p < 0.05 were considered statistically significant.

### Example 2: Intestinal steady state CX₃CR1/GFP^{hi} macrophages are replenished from monocytes and exhibit an anti-inflammatory gene expression signature

Macrophages (MΦs) are the most abundant mononuclear phagocytes in the steady state lamina propria of the colon, (**Fig 1A**), characterized by surface expression of the integrins CD11c and CD11b, as well as the F4/80 antigen. Intestinal MΦs also express unique high levels of the chemokine receptor CX₃CR1, rendering them readily detectable by flow cytometry and histology in mice harboring a GFP reporter gene insertion in their CX₃CR1 gene (Jung et al., 2000). The colonic steady state mononuclear compartment includes also CD11c^{hi}CD103⁺CD11b⁻ and to a lesser extent the CD11c⁺CD103⁺CD11b⁺ DCs (**Fig 1A****).** Adoptive monocyte transfers into MΦ-depleted animals have established that these cells are derived from Ly6C^{hi} blood monocytes (Bogunovic et al., 2009; Varol et al., 2009). Upon arrival in the tissue, monocytes are presumably by the tissue context imprinted to acquire a discrete non-inflammatory gene expression profile compatible with tissue homeostasis and their extended half-life (Rivollier et al., 2012).

To molecularly define this education process, gene expression signatures of Ly6C^{hi} monocytes and their descendants isolated from the colon of diphtheria toxin (DTx)-treated CD11c-DTR mice after monocyte engraftment and reconstitution (Varol et al., 2009) were determined. Graft-derived cells acquired a gene expression profile highly similar to CX₃CR1/GFP^{hi} resident MΦs (**Fig 1B****)** with a Pearson's correlation coefficient of 0.95 by correlation matrix analysis (**Fig 1C****).** This included induction of IL-10 and Triggering receptor expressed on myeloid cells 2 (TREM-2), IRAK-M, and TNFAIP3 (A20), but also expression of TNFα, known to exert both pro-and anti-inflammatory activities (Kassiotis and Kollias, 2001), although expression of these molecules not always reached the levels observed in CX₃CR1/GFP^{hi} resident MΦs.

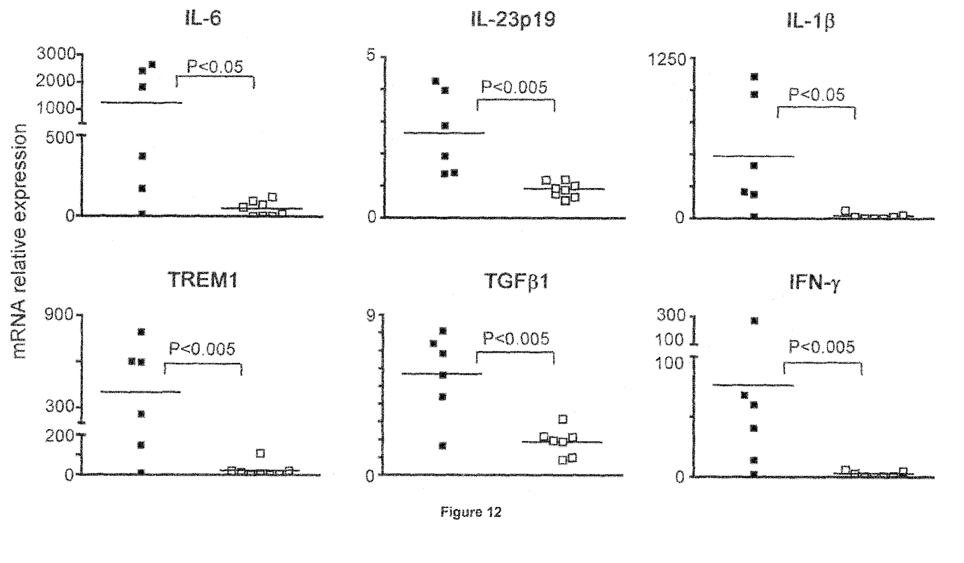

Experiments like the above have established that CX₃CR1/GFP^{hi} resident MΦs can derive from Ly6C^{hi} monocytes (Varol et al., 2009). The extended half-life of these cells (Jaensson et al., 2008; Schulz et al., 2009) however so far precluded the direct demonstration that Ly6C^{hi} monocytes differentiate in steady state *in situ* into CX₃CR1/GFP ^{hi} resident MΦs. To nevertheless study the fate of a monocyte graft in the intestinal tissue context, endoscopy-guided injection of CX₃CR1^{gfp/+} monocytes into the colonic lamina propria of healthy wt C57BL/6 mice was employed using a protocol that was recently established for an orthotopic colon cancer model (Zigmond et al., 2011). As seen in **Fig 1D****,** 12 hours after injection, Ly6C^{hi} monocytes could readily be detected as CX₃CR1/GFP^{int}Ly6C^{hi} cells in colonic cell suspensions and by 72 hours gave rise to MΦs that expressed high levels of CX₃CR1/GFP, lost Ly6C expression and acquired the F4/80 marker. Collectively, these data corroborate earlier findings (Rivollier et al., 2012; Varol et al., 2009) that Ly6C^{hi} monocytes are educated in the steady state colon to differentiate into CX₃CR1/GFP^{hi} MΦs displaying a non-inflammatory gene expression signature.

### Example 3: Intestinal inflammation induces the infiltration of CX₃CR1/GFP^{int} cells

To probe for alterations in the mononuclear phagocyte composition of the colonic lamina propria in response to acute inflammatory challenges, CX₃CR1^{gfp/+} mice were subjected to an oral dextran sodium sulfate (DSS) regimen. This established colitis model is characterized by ulceration and submucosal inflammation provoked by disruption of the epithelial barrier and resulting exposure to luminal microbiota (Okayasu et al., 1990). In accordance with earlier reports using other colitis models (Rivollier et al., 2012; Weber et al., 2011) or DSS-induced colitis (Platt et al., 2010; Waddell et al., 2011) flow cytometry analysis of the inflamed colonic lamina propria revealed the progressive emergence of CX₃CR1/GFP^{int} cells concomitant with a marked reduction in the frequency of CX₃CR1/GFP^{hi} resident MΦs (**Fig 2A**). The accumulation of CX₃CR1/GFP^{int} cells reached its peak at day 7 following the initial DSS exposure, ten-fold outnumbering CX₃CR1/GFP ^{hi} resident MΦs, and was associated with neutrophil infiltration and significant destruction of the crypt architecture, as determined by histology (**Fig 2A-C** ).Flow cytometric characterization of the CX₃CR1/GFP^{int} infiltrate identified them as CD11b^{hi} cells that expressed low levels of F4/80 and could be further divided into Ly6C^{hi} and Ly6C^{lo} subpopulations (**Fig 2D**). Of note, CX₃CR1/GFP^{int} cells are also detectable at low frequency in healthy colonic lamina propria (Platt et al., 2010; Schulz et al., 2009; Varol et al., 2009); however most of these cells were negative for the Ly6C marker (**Fig 2D**). Comparative analysis of the CX₃CR1/GFP^{int} infiltrate and the CX₃CR1/GFP^{hi} resident MΦs revealed that the former were according to their side and forward scatter smaller and less granular (**Fig 8A**). All populations expressed the pattern recognition receptor CD14, as well as the co-stimulatory molecule CD86 although expression of the latter was lower on CX₃CR1/GFP^{int} subsets. Importantly, Ly6C^{hi} CX₃CR1/GFP^{int} cells were negative for CD11c and about half of the cells expressed intermediate levels of MHC class II, while CX₃CR1/GFP ^{int}Ly6C^{lo} cells were uniformly positive for both CD11c and MHC II (**Fig 8A****, B**).

Collectively, these results show that within the acutely inflamed colon, CX₃CR1/GFP ^{hi} resident MΦs are progressively, but transiently, replaced by a heterogeneous CD11b^{hi}CX₃CR1/GFP^{int} cell population composed of a Ly6C^{hi}CD11c⁻MHCII^{-/+} and a Ly6C^{lo}CD11c⁺MHCII^{hi} cell subsets.

### Example 4: Ly6C^{hi} and Ly6C^{lo} CX₃CR1/GFP ^{int} cell subsets arise from Ly6C^{hi} monocytes under inflammatory conditions and are recruited to the gut in a CCR2-dependent manner

To probe for the origin of the CX₃CR1/GFP ^{int} cell subsets, adoptive monocyte transfers into DSS-challenged animals were performed. Rather than differentiating into CX₃CR1/GFP ^{hi} F4/80^{hi} MΦs (**Fig. 1D****),** grafted Ly6C^{hi} monocytes gave rise to CX₃CR1/GFP ^{int} Ly6C^{hi} F4/80^{lo}CD11c cells by one day after transfer. Moreover, by 72 hrs after transfer the graft had further differentiated into **CX₃CR1/GFP ^{int}** Ly6C^{lo}F4/80^{lo}cells expressing higher levels of MHCII and CD11c **(****Fig 3A**).

The chemokine receptor CCR2 is required for emigration of Ly6C^{hi} monocytes from the BM resulting in a paucity of Ly6C^{hi} monocytes in the circulation of CCR2^{-/-} mice (Serbina and Pamer, 2006). Interestingly, flow cytometry analysis of CCR2^{-/-}:CX₃CR1^{gfp/+} mice subjected to DSS-induced colitis revealed a failure of these animals to recruit both Ly6C^{hi} and accumulation of Ly6C^{lo} **CX₃CR1/GFP ^{int}** infiltrating cells to their inflamed colon, further supporting the notion that both these subsets cells derive from Ly6C^{hi} monocytes (**Fig 3B****).** Moreover, adoptive transfer of a mixed monocyte graft consisting of CD45.2 CCR2-deficient and CD45.1 CCR2-proficient CX₃CR1^{GFP/+} Ly6C^{hi} monocytes into wt recipients at day 4 after DSS challenge resulted in the exclusive recruitment of CD45.1⁺ wt cells to the inflamed colonic lamina propria (**Fig 3C**). This establishes that CCR2 is required for the recruitment of Ly6C^{hi} monocytes from the blood to the inflamed colon. Collectively, these data highlight the context-dependent fate of CCR2⁺ Ly6C^{hi} monocytes within the colonic lamina propria differentiating into CX₃CR1/GFP ^{hi} resident MΦs in steady state, but giving rise to Ly6C^{hi} and Ly6C^{lo} **CX₃CR1/GFP ^{int}** cells under inflammatory settings.

### Example 5: Molecular characterization of infiltrating CX₃CR1^{int} and resident CX₃CR1^{hi} F4/80^{hi} colonic mononuclear phagocytes

To study molecular aspects of intestinal monocyte-derived mononuclear phagocyte subsets under inflammatory conditions, an mRNA microarray analysis was performed on highly purified cells freshly isolated from the colonic lamina propria of DSS-challenged colitic CX₃CR1^{gfp/+} mice. CX₃CR1/GFP ^{hi} resident MΦs, CX₃CR1/GFP ^{int}Ly6C^{hi} cells and CX₃CR1/GFP^{int}Ly6C^{lo} cells were sorted on day 4 after DSS challenge. For comparison, CX₃CR1/GFP ^{hi} resident MΦs were isolated from untreated, healthy CX₃CR1^{gfp/+} animals (**Fig 9**). Furthermore, Ly6C^{hi} monocytes sorted from the spleens on day 4 after DSS challenge were profiled, as the splenic monocyte reservoir has been proposed to be mobilized to inflammatory foci (Swirski et al., 2009) and might hence serve as direct source of the CX₃CR1/GFP ^{int}Ly6C^{hi} cells. Microarray analysis for genes differentially expressed between the subsets revealed high similarity between CX₃CR1/GFP ^{hi} MΦs in steady state and DSS conditions (**Fig 4A****, B**), with a Pearson's correlation coefficient of 0.97 by correlation matrix analysis (**Fig 10**). The fact that this population remains largely unaffected by acute inflammation highlights the robustness of its gene expression signature. Selected gene expression analysis correlated with the markers used to sort the cells, i.e. CX₃CR1, F4/80 and Ly6C (**Fig 4B****).** While CX₃CR1/GFP^{hi} resident MΦs isolated from inflamed colons displayed the typical non-inflammatory expression pattern, CX₃CR1/GFP ^{int}Ly6C^{hi} cells exhibited a pronounced pro-inflammatory signature with high expression of TREM-1, iNOS, IL-6 and IL-23. Microarray analysis confirmed that these cells are distinct from the CX₃CR1/GFP ^{hi} resident MΦs, their Ly6C^{hi} monocytic precursors, as well as their CX₃CR1/GFP^{int}Ly6C^{lo} descendants with a Pearson's correlation coefficient of 0.82, 0.81, and 0.83, respectively (**Fig 10**). Interestingly though and as previously reported (Varol et al., 2009), the cytokine TNFα was found to be exclusively expressed by CX₃CR1/GFP ^{hi}F4/80^{hi} resident MΦs (**Fig 4B****, C**). QRT-PCR analysis of mRNA extracted from CX₃CR1/GFP ^{int} Ly6C^{hi} and Ly6C^{lo} cell subsets and resident MΦs at day 4 of DSS challenge confirmed their differential cytokine expression (**Fig 4C**).

In agreement with the role of CCR2 in its establishment (**Fig 3B****, C**), the CX₃CR1/GFP^{int}Ly6C^{hi} infiltrate uniquely expressed this chemokine receptor. The notion that CX₃CR1/GFP^{int} Ly6C^{lo} cells are CCR2^{neg}, yet significantly reduced in CCR2-deficient mice (**Fig 3B****, C**), links these cells with circulating Ly6C^{hi} monocytes and Ly6C^{hi} effector monocytes. Interestingly, the CCR2 ligands CCI2, 7 and 8 were found expressed by resident MΦs and moderately up-regulated under inflammation, potentially implying involvement of the latter in the recruitment of the monocytes (**Fig 4B****).**

Resident and infiltrating cells also differed significantly in their expression of C-type lectin receptors (CLRs) known to contribute to innate immune responses through pathogen pattern recognition (Mukhopadhyay et al., 2009). Accordingly, the DC-specific ICAM-3 grabbing non-integrin (DC-SIGN) isoform A (CD209a) was highly expressed by the infiltrating cells, while macrophage mannose receptor 1 (MRC1) expression was elevated on resident MΦs. The later also expressed higher levels of the CD163 scavenger receptor (**Fig 4B****).**

The CX₃CR1/GFP^{int}Ly6C^{lo} cells exhibited significantly lower expression of the pro-inflammatory mediators IL-6, IL-23, NOS2, VEGFa, IL-1a and TREM1 and differed considerably from the other monocyte-derived subsets by high expression of CCR7 (**Fig 4** **B,C**).

Collectively, the molecular profiles further elaborate on the phenotypic and functional variance between monocyte-derived mononuclear phagocyte subsets in steady state and inflamed colon and suggest that CX₃CR1/GFP ^{int}Ly6C^{hi} cells are pro-inflammatory effector monocytes.

### Example 6: Ablation of CX₃CR1/GFP ^{int} Ly6C^{hi} effector monocytes ameliorates DSS-induced colitis

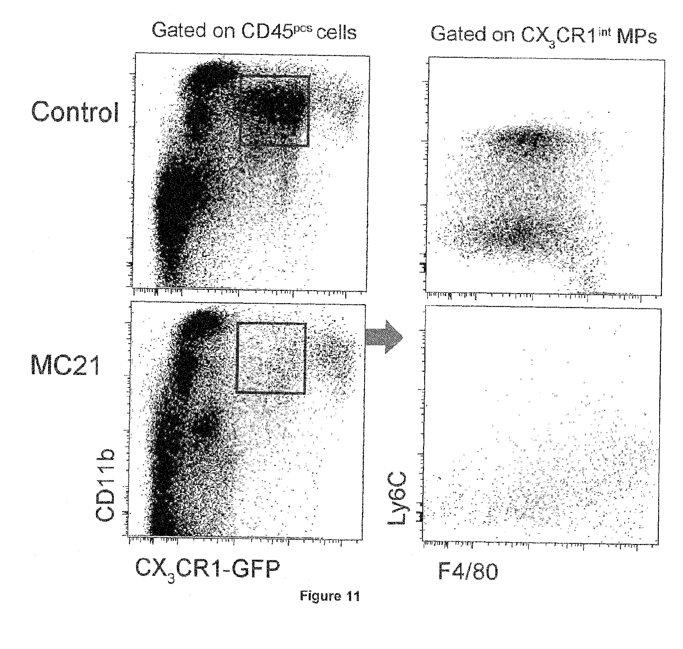

CX₃CR1/GFP ^{int}Ly6C^{hi} effector monocytes, and their immediate precursors, the Ly6C^{hi} blood monocytes, express CCR2 and are, hence, amenable to conditional *in vivo* ablation by the depleting anti-CCR2 antibody MC-21 (Bruhl et al., 2007; Shechter et al., 2009). Notably, this protocol spares CCR2^{neg} resident CX₃CR1/GFP ^{hi}F4/80^{hi} MΦs (**Fig 5A****, B**; **Fig 11**). To investigate the functional contribution of the CX₃CR1/GFP ^{int}Ly6C^{hi} effector monocytes to the DSS-induced gut inflammation, CX₃CR1^{gfp/+} animals were injected with MC21 starting at day 2 after DSS exposure, once a day, for a period of 5 days, i.e. throughout the window of progressive colonic recruitment of Ly6C^{hi} monocytes (**Fig 2A-C** ).Flow cytometry analysis of blood of the MC21-treated animals confirmed the depletion of Ly6C^{hi}, but not CCR2⁻ Ly6C^{lo} monocytes (**Fig 5A**). Analysis of the colonic lamina propria revealed the absence of CX₃CR1/GFP ^{int}Ly6C^{hi} effector monocytes. Importantly, no alterations of resident MΦs and neutrophil frequencies were observed, while also CCR2^{neg} CX₃CR1/GF^{int}Ly6C^{lo} cells were significantly reduced supporting their origin from CCR2⁺ effector monocytes (**Fig 5A****, B,** **Fig 11**). Strikingly, DSS challenged mice depleted of Ly6C^{hi} blood monocytes and the CX₃CR1/GFP^{int} infiltrate exhibited significantly milder features of colitis as evaluated and quantified by colonoscopy and body mass change at day 7 after DSS onset (**Fig 5C-E**). Moreover, ELISA on colon explant cultures showed a significant reduction of pro-inflammatory cytokines, such as IL-6 and IL-1β, as well as IFNγ (**Fig 5F**). This finding was corroborated by results of a qRT-PCR analysis of mRNA extracted from the colonic tissue which revealed significantly reduced levels of IL-6, IL-23p19, IL-1α, IL1-β, TGFβ1 and TREM1, all shown by chip analysis to be expressed in high levels exclusively in effector monocytes, alongside with reduced IFNγ expression (**Fig 12**).

Collectively these data establish CX₃CR1/GFP^{int}Ly6C^{hi} effector monocytes in this acute experimental colitis model as pivotal drivers of the colonic inflammation.

### Example 7: Pro-inflammatory activities of CX₃CR1/GFP ^{int} Ly6C^{hi} effector monocytes can be triggered by TLR2 / NOD2 ligands

A comprehensive '*function and pathway*' analysis of the microarray data revealed the selective activation of Nucleotide-binding oligomerization domain-containing protein (NOD) like receptor signaling pathway in CX₃CR1/GFP ^{int} Ly6C^{hi} effector monocytes vs. resident CX₃CR1/GFP^{hi} MΦs and CX₃CR1/GFP ^{int} Ly6C^{lo} cells. More specifically, the array data and subsequent qRT-PCR analysis showed high mRNA expression levels of NOD1/2, Caspase recruitment domain-containing protein 9 (CARD 9), as well as increased expression of Toll-like receptor 2 (TLR2), Inhibitor of nuclear factor kappa-B kinase subunit beta (IKK-β or IKBKB) and the NF-kappa-B complex proteins ReIB, NFKB1 (p50) and NFKB2 (p52) in infiltrating compared to resident cells (**Fig 6A****, B**). Importantly, TLR2 and NOD2 expression were also up-regulated in effector monocytes, as compared to splenic Ly6C^{hi} monocytes (**Fig 6A**).

To probe whether the pro-inflammatory signature of the effector monocytes could have been a result of TLR and NOD-2 engagement, the cells were isolated from colitic mice and exposed to the respective ligands. CX₃CR1/GFP^{int}Ly6C^{hi} effector monocytes treated with the synthetic TLR2 ligand Pam3CSK4 but also the TLR4 ligand LPS responded to the stimuli with substantial IL-6 secretion (**Fig 6C**), well exceeding the response of CX₃CR1/GFP^{int}Ly6C^{lo} cells and CX₃CR1/GFP^{hi} resident MΦs, included in this assay. Addition of muramyl dipeptide (MDP), a peptidoglycan constituent of Gram positive and - negative bacteria and ligand for the intracellular sensor NOD2, resulted in synergistic augmentation of IL-6 production.

To test the role of TLR2, NOD2 in the development of acute gut inflammation, BM chimeras were generated with the respective mutant BM. After exposure of the animals to DSS, [TLR2^{-/-} > wt] and [NOD2^{-/-} > wt] chimeras exhibited significantly milder features of colitis as assessed and quantified by colonoscopy at day 7 after onset of DSS application (**Fig 6D****, E**). Moreover, in support of the notion of an exacerbating role of microbial product-sensing innate immune cells in the DSS model, also [Myd88^{-/-} Trif^{-/-} > wt] mice showed a relative protection as compared to [wt > wt] controls (**Fig 6D****, E**). Expression of IL-6 and IL23 (p19) was found only moderately reduced in TLR2^{-/-} and NOD2^{-/-} Ly6C^{hi} effector monocytes compared to wt cells, but most evident in Myd88^{-/-} Trif^{-/-} Ly6C^{hi} effector monocytes (**Fig 6F**). Thus, TLRs other than TLR2 might also be critical for induction of pro-inflammatory cytokines in CX₃CR1/GFP^{int}Ly6C^{hi} effector monocytes.

Collectively, these results suggest that the pro-inflammatory signature of CX₃CR1/GFP^{int}Ly6C^{hi} effector monocytes results from their exposure to bacterial products in the lamina propria and the activation of the TLR and NOD2 pathways in these cells.

### Example 8: Monocyte-derived CX₃CR1/GFP ^{int}Ly6C^{lo} cells are migratory, acquire and process luminal antigens and can stimulate T cells

The prominent hallmarks of classical DCs are their ability to migrate to the tissue draining LNs and stimulate naïve T cells. In the intestinal lamina propria, CCR7 dependent migration to the mLNs has been shown to be specific feature of CD103⁺ lamina propria DCs (Bogunovic et al., 2009; Coombes et al., 2007; Jaensson et al., 2008; Johansson-Lindbom et al., 2005; Schulz et al., 2009), while CCR7-negative CX₃CR1/GFP ^{hi}F4/80^{hi} resident MΦs are considered non-migratory and hence absent from lymph and mesenteric LNs (Schulz et al., 2009). The adoptive transfer established that in inflamed colon, Ly6C^{hi} monocytes give sequential rise to CX₃CR1/GFP^{int}Ly6C^{hi} and CX₃CR1/GFP^{int}Ly6C^{lo} cells (**Fig 3A**). Interestingly, the microarray analysis revealed significant CCR7 mRNA expression in Ly6C^{hi} effector monocytes as compared to CX₃CR1/GFP ^{hi}F4/80^{hl} resident MΦs and Ly6C^{hi} splenic monocytes. Moreover, CCR7 transcription was further up-regulated in CX₃CR1/GFP^{int}Ly6C^{lo} cells (**Fig 4B****),** as also confirmed by qRT-PCR analysis (**Fig 7A**). CX₃CR1/GFP^{int}Ly6C^{lo} cells did not respond to the Pam3/MDP challenge with IL-6 production and thus seem to have lost the pro-inflammatory activity of effector monocytes (**Fig 6C**). In order to examine whether CCR7 expression on CX₃CR1/GFP^{int} monocyte-derived cells endows these cells with a capability to migrate *in vivo,* two photon microscopy imaging was employed. To visualize afferent lymphatic vessels draining the proximal colon towards the mesenteric LNs and distinguish them from blood vessels, mice were i.v. injected with Evans Blue followed by injection of Dextran-FITC 30 minutes later. This resulted in lymphatic vessels labeled with the drained Evans Blue fluorescence (red), while blood vessels lit up in both red and green (**Fig 7B****).** In agreement with earlier reports on the small intestinal lymphatics (Schulz et al., 2009), scanning of the colonic lymphatic vasculature of CX₃CR1^{gfp/+} mice in steady state demonstrated only rare CX₃CR1/GFP⁺ cells inside the vessels (**Fig 7B****).** In contrast, analysis of lymphatics draining the colon of DSS-challenged mice revealed numerous CX₃CR1/GFP⁺ cells in the vessel lumen (**Fig 7B****),** supporting the notion that CCR7⁺ CX₃CR1/GFP^{int} monocyte-derived cells are migratory.

In light of the transfer results (**Fig 3A**), these data suggest that the monocyte graft gives sequential rise to Ly6C^{hi} effector monocytes and migratory Ly6C^{lo} cells that up-regulate MHC II expression and hence seem to acquire APC potential. To directly address this point, the ability of the various monocyte-derived mononuclear phagocyte subsets to ingest and process orally applied antigen for naïve T cell stimulation was next tested. DSS-challenged colitic CX₃CR1^{gfp/+} mice were gavaged with ovalbumin (OVA) protein and 12 hrs later sacrificed to isolate colonic CX₃CR1/GFP^{int}Ly6C^{hi} effector monocytes, CX₃CR1/GFP^{int}Ly6C^{lo} cells and CX₃CR1/GFP^{hi} resident MΦs for co-culture with CFSE labeled OVA-specific CD4⁺ T cells (OT II cells). As shown in **Fig 7C**, CX₃CR1/GFP^{int}Ly6C^{lo} cells induced a significant proliferative expansion of the CD4⁺ T cells as indicated by the dilution of the CFSE dye (and TCR down-modulation). In contrast, the CX₃CR1/GFP^{int}Ly6C^{hi} cells exhibited only moderate antigen stimulatory capability, and, in accordance with previous studies (Schulz et al., 2009), CX₃CR1/GFP^{hi} F4/80^{hi} resident MΦs failed to prime the naïve CD4⁺ T cells altogether.

Besides their high MHC II and CCR7 expression, the gene expression analysis of CX₃CR1/GFP^{int}Ly6C^{lo} cells also revealed other DC signatures, such as expression of CD135, the receptor for the cytokine Fms-related tyrosine kinase 3 ligand (Flt3L) and expression of transcription factor Zbtb46 (BTBD4), a member of the BTB-ZF family, recently proposed to be specifically expressed in cDCs among immune cells (Meredith et al., 2012; Satpathy et al., 2012). To further address the Zbtb46 expression within the CX₃CR1/GFP^{int}Ly6C^{lo} cell compartment, irradiated wt recipients were reconstituted with BM obtained from *zbtb46*^{gfp/+} mice (Satpathy et al., 2012) and the [*zbtb46*^{gfp/+} > wt] mice were subjected to a DSS challenge. DSS-treated [*zbtb46*^{gfp/+} > wt] mice displayed a prominent accumulation of Ly6C^{lo} cells - a fraction of which expressed the GFP label thus confirming Zbtb46 expression. Moreover, treatment of the mice with the CCR2-antibody significantly reduced both Zbtb46/GFP⁺ and Zbtb46/GFP⁻ Ly6C^{lo} cells, confirming that both cell types were recently derived from CCR2-expressing cells.

Collectively, these results suggest that monocytes that enter the inflamed colon differentiate with time into migratory antigen presenting cells exhibiting phenotypic and functional characteristics of monocyte-derived DCs.

### Example 9: Production of antibodies against human CCR2 and depletion of CCR2-positive monocytes using these antibodies

For the production of antibodies against human CCR2, BALB/c-mice that were at least 6 weeks old, were immunized intraperitoneally 6-8 times in intervals of at least 3 weeks with at least 10 million CHO cells stably transfected with full-length CCR2b. Four days after the last immunization the spleen cells were fused with X63.Ag8.653 myeloma cells and cultivated in selective medium. The supernatant of the thus generated hybridomas was tested by means of FACS analysis for the binding to CCR2b-transfected or, as a control, for the binding to CXCR4-transfected CHO cells, respectively. After positive cultures were re-cloned twice, several monoclonal antibodies were obtained which specifically bind to human CCR2 and neither show a cross-reaction with murine CCR2 or CCR5 nor with human CCR1, CCR3, CCR5 or CXCR4. In human peripheral blood, the antibodies bound to a sub-population of the T cells (about 20-30%), as well as to the predominant number of the CD14+ monocytes (>95%). By preincubation of the human cells with MCP-1 (1 µg/ml) for 30 min at 37 °C, the binding of the CCR2 antibodies to the leukocyte sub-populations described above could virtually completely be inhibited, which can be explained by a MCP-1-induced internalization of CCR2. This additionally showed the specificity of the antibodies.

For the determination of the binding epitopes of the antibodies, the binding to cells expressing chimeric receptors consisting of CCR2 and CCR5 was determined. The internalization (down-modulation) of CCR2 was also measured by means of flow cytometry.

In addition, it was examined whether primates exhibit a cross-reaction with antibodies against human CCR2. The cross-reaction of the three antibodies with CCR2 on leukocytes of common marmosets (Callithrix jacchus) was examined by means of FACS analysis. For that, whole-blood of common marmosets was incubated with 5 µg/ml of the CCR2 antibody or with the same concentration of isotype control antibodies (IgG1 and IgG3, respectively), which was followed by a PE (phycoerythrin)-labeled secondary antibody (Rabbit (F(ab)2 fragment anti-mouse lg, R0439, DakoCytomation). After lysis of the erythrocytes, the FACS analysis was carried out.

In order to additionally prove the specificity of binding of the antibodies to common marmoset CCR2, for a part of the whole blood, an internalization of CCR2 was induced with human MCP-1 before the incubation with the CCR2 antibodies. For this, the whole blood was pre-incubated with 1 µg/ml MCP-1 for 30 min at 37 °C and was subsequently examined for the binding of the CCR2 antibodies as described above. The strong binding of two of the antibodies and a slightly weaker binding of the third antibody was shown on the monocyte population which is to be delineated in the forward-sideward scatter of the FACS dot plot. About 50% of the thus delineated monocytes bound to the antibodies. This binding was completely lost when MCP-1-pre-incubated leukocytes were used. The isotype control antibodies did not show any binding to monocytes. Apart from the monocyte sub-population, a binding of the CCR2 antibodies only occurred to a very small further leukocyte sub-population, which is to be classified as basophilic granulocytes due to its forward-sideward scatter. The monocyte population recognized by the CCR2 antibodies homogenously was CD11b positive, which was shown by staining with the antibody clone (M1/70, rat anti-mouse CD11b). This CD11b antibody showed a cross-reaction with human and also with common marmoset CD11b.

In addition, the pharmacokinetic features of two of the CCR2 antibodies were examined. 24, 48 and 96 hours after injection of the antibodies (5 mg/kg body weight), the plasma concentrations of the antibodies were measured. For this, plasma of the monkeys was incubated in various dilutions with hCCR2b-transfected CHO cells, and the plasma concentration of the CCR2 antibody was calculated by means of a standard curve consisting of different dilutions of the antibodies.

Moreover, it was examined whether a depletion of the CCR2-positive monocytes occurred after injection of CCR2 antibodies. For that, blood was taken 24 hours, 48 hours and 96 hours after injection of the CCR2 antibodies and an isotype control antibody, respectively (5 mg/kg body weight each, 2 monkeys per group) and the proportion of CCR2-positive monocytes of the total number of leukocytes was determined by means of FACS analysis. For this purpose, whole-blood was stained with the CCR2 antibodies, followed by a phycoerythrin-labeled secondary antibody as described above, and the CCR2-positive monocytes were identified in connection with the forward-sideward scatter. The proportion of the CCR2-positive monocytes of the total number of leukocytes, on the first day after injection, was reduced in the animals treated with the CCR2 antibodies (to about 50%). The proportion of the CCR2-positive monocytes of the total number of leukocytes in the group treated with isotype control antibodies at the respective days was set to be 100%.

### References

Ajami, B., Bennett, J.L., Krieger, C., McNagny, K.M., and Rossi, F.M. (2011). Infiltrating monocytes trigger EAE progression, but do not contribute to the resident microglia pool. Nat Neurosci 14, 1142-1149.
Artis, D. (2008). Epithelial-cell recognition of commensal bacteria and maintenance of immune homeostasis in the gut. Nat Rev Immunol 8, 411-420.
Atarashi, K., Tanoue, T., Shima, T., Imaoka, A., Kuwahara, T., Momose, Y., Cheng, G., Yamasaki, S., Saito, T., Ohba, Y., et al. (2011). Induction of colonic regulatory T cells by indigenous Clostridium species. Science 331, 337-341.
Auffray, C., Fogg, D., Garfa, M., Elain, G., Join-Lambert, O., Kayal, S., Sarnacki, S., Cumano, A., Lauvau, G., and Geissmann, F. (2007). Monitoring of blood vessels and tissues by a population of monocytes with patrolling behavior. Science 317, 666-670.
Barnden, M.J., Allison, J., Heath, W.R., and Carbone, F.R. (1998). Defective TCR expression in transgenic mice constructed using cDNA-based alpha- and beta-chain genes under the control of heterologous regulatory elements. Immunol Cell Biol 76, 34-40.
Bevins, C.L., and Salzman, N.H. (2011). Paneth cells, antimicrobial peptides and maintenance of intestinal homeostasis. Nat Rev Microbiol 9, 356-368.
Biswas, S.K., and Mantovani, A. (2010). Macrophage plasticity and interaction with lymphocyte subsets: cancer as a paradigm. Nat Immunol 11, 889-896.
Bogunovic, M., Ginhoux, F., Helft, J., Shang, L., Hashimoto, D., Greter, M., Liu, K., Jakubzick, C., Ingersoll, M.A., Leboeuf, M., et al. (2009). Origin of the lamina propria dendritic cell network. Immunity 31, 513-525.
Bruhl, H., Cihak, J., Plachy, J., Kunz-Schughart, L., Niedermeier, M., Denzel, A., Rodriguez Gomez, M., Talke, Y., Luckow, B., Stangassinger, M., and Mack, M. (2007). Targeting of Gr-1+,CCR2+ monocytes in collagen-induced arthritis. Arthritis Rheum 56, 2975-2985.
Chande et al., Interventions for treating collagenous colitis; Cochrane Database Syst Rev. 2006 Oct 18;(4).
Charo IF, Myers SJ, Herman A, Franci C, Connolly AJ, Coughlin SR. Molecular cloning and functional expression of two monocyte chemoattractant protein 1 receptors reveals alternative splicing of the carboxyl-terminal tails. Proc Natl Acad Sci USA. 1994 Mar 29;91(7):2752-6.
Cheong, C., Matos, I., Choi, J.H., Dandamudi, D.B., Shrestha, E., Longhi, M.P., Jeffrey, K.L.. Anthony, R.M., Kluger, C., Nchinda, G., et al. (2010). Microbial stimulation fully differentiates monocytes to DC-SIGN/CD209(+) dendritic cells for immune T cell areas. Cell 143, 416-429.
Cho, J.H. (2008). The genetics and immunopathogenesis of inflammatory bowel disease. Nat Rev Immunol 8, 458-466.
Coombes, J.L., Siddiqui, K.R., Arancibia-Carcamo, C.V., Hall, J., Sun, C.M., Belkaid, Y., and Powrie, F. (2007). A functionally specialized population of mucosal CD103+ DCs induces Foxp3+ regulatory T cells via a TGF-beta and retinoic acid-dependent mechanism. J Exp Med 204, 1757-1764.
Cros, J., Cagnard, N., Woollard, K., Patey, N., Zhang, S.Y., Senechal, B., Puel, A., Biswas, S.K., Moshous, D., Picard, C., et al. (2010). Human CD14dim monocytes patrol and sense nucleic acids and viruses via TLR7 and TLR8 receptors. Immunity 33, 375-386.
Denning, T.L., Wang, Y.C., Patel, S.R., Williams, I.R., and Pulendran, B. (2007). Lamina propria macrophages and dendritic cells differentially induce regulatory and interleukin 17-producing T cell responses. Nat Immunol 8, 1086-1094.
Doyon J, Coesemans E, Boeckx S, Buntinx M, Hermans B, Van Wauwe JP, Gilissen RA, De Groot AH, Corens D, Van Lommen G, Discovery of potent, orally bioavailable small-molecule inhibitors of the human CCR2 receptor. ChemMedChem. 2008 Apr;3(4):660-9.
Edelson, B.T., Kc, W., Juang, R., Kohyama, M., Benoit, L.A., Klekotka, P.A., Moon, C., Albring, J.C., Ise, W., Michael, D.G., et al. (2010). Peripheral CD103+ dendritic cells form a unified subset developmentally related to CD8alpha+ conventional dendritic cells. J Exp Med 207, 823-836.
Feigenbaum, Description of Behçet's syndrome in the Hippocratic third book of endemic diseases. Br J Ophthalmol 1956; 40:355
Ford AC, Sandborn WJ, Khan KJ, Hanauer SB, Talley NJ, Moayyedi P. Efficacy of biological therapies in inflammatory bowel disease: systematic review and meta-analysis. Am J Gastroenterol. 2011 Apr;106(4):644-59, quiz 660. Epub 2011 Mar 15.
Garud and Peppercorn, Ulcerative colitis: current treatment strategies and future prospects; Therapeutic Advances in Gastroenterology March 2009 2: 99-108
Geissmann, F., Jung, S., and Littman, D.R. (2003). Blood monocytes consist of two principal subsets with distinct migratory properties. Immunity 19, 71-82.
George J, Present DH, Pou R, Bodian C, Rubin PH. The long-term outcome of ulcerative colitis treated with 6-mercaptopurine. Am J Gastroenterol. 1996 Sep;91 (9):1711-4.;
Grimm, M.C., Pullman, W.E., Bennett, G.M., Sullivan, P.J., Pavli, P., and Doe, W.F. (1995). Direct evidence of monocyte recruitment to inflammatory bowel disease mucosa. J Gastroenterol Hepatol 10, 387-395.
Hadis, U., Wahl, B., Schulz, O., Hardtke-Wolenski, M., Schippers, A., Wagner, N., Muller, W., Sparwasser, T., Forster, R., and Pabst, O. (2011). Intestinal tolerance requires gut homing and expansion of FoxP3+ regulatory T cells in the lamina propria. Immunity 34, 237-246.
Hashimoto, D., Miller, J., and Merad, M. (2011). Dendritic cell and macrophage heterogeneity in vivo. Immunity 35, 323-335.
Higgins Paul J., Schwartz C. Eric und Nicolas Jean-Marie, Small molecule CCR2 antagonists. Chemokine Biology - Basic Research and Clinical Application; Progress in Inflammation Research, 2007, Part 2, 115-123.
Irizarry, R.A., Hobbs, B., Collin, F., Beazer-Barclay, Y.D., Antonellis, K.J., Scherf, U., and Speed, T.P. (2003). Exploration, normalization, and summaries of high density oligonucleotide array probe level data. Biostatistics 4, 249-264.
Ivanov, II, Atarashi, K., Manel, N., Brodie, E.L., Shima, T., Karaoz, U., Wei, D., Goldfarb, K.C., Santee, C.A., Lynch, S.V., et al. (2009). Induction of Intestinal Th17 Cells by Segmented Filamentous Bacteria. Cell.
Jaensson, E., Uronen-Hansson, H., Pabst, O., Eksteen, B., Tian, J., Coombes, J.L., Berg, P.L., Davidsson, T., Powrie, F., Johansson-Lindbom, B., and Agace, W.W. (2008). Small intestinal CD103+ dendritic cells display unique functional properties that are conserved between mice and humans. J Exp Med 205, 2139-2149.
Johansson-Lindbom, B., Svensson, M., Pabst, O., Palmqvist, C., Marquez, G., Forster, R., and Agace, W.W. (2005). Functional specialization of gut CD103+ dendritic cells in the regulation of tissue-selective T cell homing. J Exp Med 202, 1063-1073.
Jung, S., Aliberti, J., Graemmel, P., Sunshine, M.J., Kreutzberg, G.W., Sher, A., and Littman, D.R. (2000). Analysis of fractalkine receptor CX(3)CR1 function by targeted deletion and green fluorescent protein reporter gene insertion. Mol Cell Biol 20, 4106-4114.
Jung, S., Unutmaz, D., Wong, P., Sano, G., De los Santos, K., Sparwasser, T., Wu, S., Vuthoori, S., Ko, K., Zavala, F., et al. (2002). In vivo depletion of CD11c(+) dendritic cells abrogates priming of CD8(+) T cells by exogenous cell-associated antigens. Immunity. 17, 211-220.
Kamada, N., Hisamatsu, T., Okamoto, S., Chinen, H., Kobayashi, T., Sato, T., Sakuraba, A., Kitazume, M.T., Sugita, A., Koganei, K., et al. (2008). Unique CD14 intestinal macrophages contribute to the pathogenesis of Crohn disease via IL-23/IFN-gamma axis. J Clin Invest 118, 2269-2280.
Kassiotis, G., and Kollias, G. (2001). Uncoupling the proinflammatory from the immunosuppressive properties of tumor necrosis factor (TNF) at the p55 TNF receptor level: implications for pathogenesis and therapy of autoimmune demyelination. J Exp Med 193, 427-434.
Kobayashi, K.S., Chamaillard, M., Ogura, Y., Henegariu, O., Inohara, N., Nunez, G., and Flavell, R.A. (2005). Nod2-dependent regulation of innate and adaptive immunity in the intestinal tract. Science 307, 731-734.
Lichtiger S, Present DH, Kornbluth A, Gelernt I, Bauer J, Galler G, Michelassi F, Hanauer S. Cyclosporine in severe ulcerative colitis refractory to steroid therapy. N Engl J Med. 1994 Jun 30;330(26):1841-5.
Lin, M.L., Zhan, Y., Proietto, A.I., Prato, S., Wu, L., Heath, W.R., Villadangos, J.A., and Lew, A.M. (2008). Selective suicide of cross-presenting CD8+ dendritic cells by cytochrome c injection shows functional heterogeneity within this subset. Proc Natl Acad Sci U S A 105, 3029-3034.
Liu, K., Victora, G.D., Schwickert, T.A., Guermonprez, P., Meredith, M.M., Yao, K., Chu, F.F., Randolph, G.J., Rudensky, A.Y., and Nussenzweig, M. (2009). In vivo analysis of dendritic cell development and homeostasis. Science 324, 392-397.
Marshall JK, Irvine EJ., Rectal corticosteroids versus alternative treatments in ulcerative colitis: a meta-analysis. Gut. 1997 Jun;40(6):775-81.
Mercolino TJ, Connelly MC, Meyer EJ, Knight MD, Parker JW, Stelzer GT, DeChirico G. Immunologic differentiation of absolute lymphocyte count with an integrated flow cytometric system: a new concept for absolute T cell subset determinations. Cytometry. 1995 Mar 15;22(1):48-59.
Meredith, M.M., Liu, K., Darrasse-Jeze, G., Kamphorst, A.O., Schreiber, H.A., Guermonprez, P., Idoyaga, J., Cheong, C., Yao, K.H., Niec, R.E., and Nussenzweig, M.C. (2012). Expression of the zinc finger transcription factor zDC (Zbtb46, Btbd4) defines the classical dendritic cell lineage. J Exp Med 209, 1153-1165.
Mukhopadhyay, S., Pluddemann, A., and Gordon, S. (2009). Macrophage pattern recognition receptors in immunity, homeostasis and self tolerance. Adv Exp Med Biol 653, 1-14.
Murai, M., Turovskaya, O., Kim, G., Madan, R., Karp, C.L., Cheroutre, H., and Kronenberg, M. (2009). Interleukin 10 acts on regulatory T cells to maintain expression of the transcription factor Foxp3 and suppressive function in mice with colitis. Nat Immunol 10, 1178-1184.
Niess, J.H., and Adler, G. Enteric flora expands gut lamina propria CX3CR1+ dendritic cells supporting inflammatory immune responses under normal and inflammatory conditions. J Immunol 184, 2026-2037.
Niess, J.H., Brand, S., Gu, X., Landsman, L., Jung, S., McCormick, B.A., Vyas, J.M., Boes, M., Ploegh, H.L., Fox, J.G., et al. (2005). CX3CR1-mediated dendritic cell access to the intestinal lumen and bacterial clearance. Science 307, 254-258.
Okayasu, I., Hatakeyama, S., Yamada, M., Ohkusa, T., Inagaki, Y., and Nakaya, R. (1990). A novel method in the induction of reliable experimental acute and chronic ulcerative colitis in mice. Gastroenterology 98, 694-702.
Palframan, R.T., Jung, S., Cheng, G., Weninger, W., Luo, Y., Dorf, M., Littman, D.R., Rollins, B.J., Zweerink, H., Rot, A., and von Andrian, U.H. (2001). Inflammatory chemokine transport and presentation in HEV: a remote control mechanism for monocyte recruitment to lymph nodes in inflamed tissues. J Exp Med 194, 1361-1373.
Pearson DC, May GR, Fick GH, Sutherland LR. Azathioprine and 6-mercaptopurine in Crohn disease. A meta-analysis. Ann Intern Med. 1995 Jul 15;123(2):132-42.
Peterson, D.A., McNulty, N.P., Guruge, J.L., and Gordon, J.1. (2007). IgA response to symbiotic bacteria as a mediator of gut homeostasis. Cell Host Microbe 2, 328-339.
Platt, A.M., Bain, C.C., Bordon, Y., Sester, D.P., and Mowat, A.M. (2010). An independent subset of TLR expressing CCR2-dependent macrophages promotes colonic inflammation. J Immunol 184, 6843-6854.
Powrie, F., Leach, M.W., Mauze, S., Caddle, L.B., and Coffman, R.L. (1993). Phenotypically distinct subsets of CD4+ T cells induce or protect from chronic intestinal inflammation in C. B-17 scid mice. Int Immunol 5, 1461-1471.
Rahimi R, Nikfar S, Rezaie A, Abdollahi M.; A meta-analysis of antibiotic therapy for active ulcerative colitis. Dig Dis Sci. 2007 Nov;52(11):2920-5. Epub 2007 Apr 6.
Rescigno, M., Urbano, M., Valzasina, B., Francolini, M., Rotta, G., Bonasio, R., Granucci, F., Kraehenbuhl, J.P., and Ricciardi-Castagnoli, P. (2001). Dendritic cells express tight junction proteins and penetrate gut epithelial monolayers to sample bacteria. Nat Immunol 2, 361-367.
Riley SA, Mani V, Goodman MJ, Herd ME, Dutt S, Turnberg LA.; Comparison of delayed release 5 aminosalicylic acid (mesalazine) and sulphasalazine in the treatment of mild to moderate ulcerative colitis relapse. Gut. 1988 May;29(5):669-74.
Rivollier, A., He, J., Kole, A., Valatas, V., and Kelsall, B.L. (2012). Inflammation switches the differentiation program of Ly6Chi monocytes from antiinflammatory macrophages to inflammatory dendritic cells in the colon. J Exp Med 209, 139-155.
Rostom et al., Celiac disease, Evid Rep Technol Assess (Summ). 2004 Jun;(104):1-6
Rydstrom, A., and Wick, M.J. (2010). Salmonella inhibits monocyte differentiation into CD11c hi MHC-II hi cells in a MyD88-dependent fashion. J Leukoc Biol 87, 823-832.
Satpathy, A.T., Kc, W., Albring, J.C., Edelson, B.T., Kretzer, N.M., Bhattacharya, D., Murphy, T.L., and Murphy, K.M. (2012). Zbtb46 expression distinguishes classical dendritic cells and their committed progenitors from other immune lineages. J Exp Med 209, 1135-1152.
Saunders, D., Lucas, K., Ismaili, J., Wu, L., Maraskovsky, E., Dunn, A., and Shortman, K. (1996). Dendritic cell development in culture from thymic precursor cells in the absence of granulocyte/macrophage colony-stimulating factor. J Exp Med 184, 2185-2196.
Schenk, M., Bouchon, A., Seibold, F., and Mueller, C. (2007). TREM-1--expressing intestinal macrophages crucially amplify chronic inflammation in experimental colitis and inflammatory bowel diseases. J Clin Invest 117, 3097-3106.
Schulz, C., Gomez Perdiguero, E., Chorro, L., Szabo-Rogers, H., Cagnard, N., Kierdorf, K., Prinz, M., Wu, B., Jacobsen, S.E., Pollard, J.W., et al. (2012). A lineage of myeloid cells independent of Myb and hematopoietic stem cells. Science 336, 86-90.
Schulz, O., Jaensson, E., Persson, E.K., Liu, X., Worbs, T., Agace, W.W., and Pabst, O. (2009). Intestinal CD103+, but not CX3CR1+, antigen sampling cells migrate in lymph and serve classical dendritic cell functions. J Exp Med 206, 3101-3114.
Serbina, N.V., and Pamer, E.G. (2006). Monocyte emigration from bone marrow during bacterial infection requires signals mediated by chemokine receptor CCR2. Nat Immunol 7, 311-317.
Serbina, N.V., Salazar-Mather, T.P., Biron, C.A., Kuziel, W.A., and Pamer, E.G. (2003). TNF/iNOS-producing dendritic cells mediate innate immune defense against bacterial infection. Immunity 19,59-70.
Shin Niu, Baribaud Frédéric, Wang Kathy, Yang Genjie, Wynn Rich, Covington Maryanne B., Feldman Patricia, Gallagher Karen B., Leffet Lynn M., Lo Yvonne Y., Wang Anlai, Xue Chu-Biao, Newton Robert C., Scherle Peggy A., Pharmacological characterization of INCB3344, a small molecule antagonist of human CCR2, Biochemical and Biophysical Research Communications, Volume 387, Issue 2, 18 September 2009, Pages 251-255
Shechter, R., London, A., Varol, C., Raposo, C., Cusimano, M., Yovel, G., Rolls, A., Mack, M., Pluchino, S., Martino, G., et al. (2009). Infiltrating blood-derived macrophages are vital cells playing an anti-inflammatory role in recovery from spinal cord injury in mice. PLoS Med 6, e1000113.
Siddiqui, K.R., Laffont, S., and Powrie, F. (2010). E-cadherin marks a subset of inflammatory dendritic cells that promote T cell-mediated colitis. Immunity 32, 557-567.
Smythies, L.E., Sellers, M., Clements, R.H., Mosteller-Barnum, M., Meng, G., Benjamin, W.H., Orenstein, J.M., and Smith, P.D. (2005). Human intestinal macrophages display profound inflammatory anergy despite avid phagocytic and bacteriocidal activity. J Clin Invest 115, 66-75.
Sun, C.M., Hall, J.A., Blank, R.B., Bouladoux, N., Oukka, M., Mora, J.R., and Belkaid, Y. (2007). Small intestine lamina propria dendritic cells promote de novo generation of Foxp3 T reg cells via retinoic acid. J Exp Med 204, 1775-1785.
Swirski, F.K., Nahrendorf, M., Etzrodt, M., Wildgruber, M., Cortez-Retamozo, V., Panizzi, P., Figueiredo, J.L., Kohler, R.H., Chudnovskiy, A., Waterman, P., et al. (2009). Identification of splenic reservoir monocytes and their deployment to inflammatory sites. Science 325, 612-616.
Szczepkowski et al. Acta Chir lugosl. 2008;55(3):77-81
Temmerman and Baert, Collagenous and lymphocytic colitis: systematic review and update of the literature; Dig Dis. 2009;27 Suppl 1:137-45.
Travassos, L.H., Girardin, S.E., Philpott, D.J., Blanot, D., Nahori, M.A., Werts, C., and Boneca, I.G. (2004). Toll-like receptor 2-dependent bacterial sensing does not occur via peptidoglycan recognition. EMBO Rep 5, 1000-1006.
Turner D, Walsh CM, Steinhart AH, Griffiths AM.; Response to corticosteroids in severe ulcerative colitis: a systematic review of the literature and a meta-regression. Clin Gastroenterol Hepatol. 2007 Jan;5(1):103-10. Epub 2006 Dec 4.
Vangoitsenhoven et al., Whipple's disease. A classic case report and review of the literature, Acta Gastroenterol Belg. 2010 Jul-Sep;73(3):392-6.
Varol, C., Landsman, L., Fogg, D.K., Greenshtein, L., Gildor, B., Margalit, R., Kalchenko, V., Geissmann, F., and Jung, S. (2007). Monocytes give rise to mucosal, but not splenic, conventional dendritic cells. J Exp Med 204, 171-180.
Varol, C., Vallon-Eberhard, A., Elinav, E., Aychek, T., Shapira, Y., Luche, H., Fehling, H.J., Hardt, W.D., Shakhar, G., and Jung, S. (2009). Intestinal lamina propria dendritic cell subsets have different origin and functions. Immunity 31, 502-512.
Varol, C., Zigmond, E., and Jung, S. (2010). Securing the immune tightrope: mononuclear phagocytes in the intestinal lamina propria. Nat Rev Immunol 10, 415-426.
Vermeire S, Van Assche G, Rutgeerts P. Classification of inflammatory bowel disease: the old and the new. Curr Opin Gastroenterol. 2012 Jul;28(4):321-6. Review.
Waddell, A., Ahrens, R., Steinbrecher, K., Donovan, B., Rothenberg, M.E., Munitz, A., and Hogan, S.P. (2011). Colonic eosinophilic inflammation in experimental colitis is mediated by Ly6C(high) CCR2(+) inflammatory monocyte/macrophage-derived CCL11. J Immunol 186, 5993-6003.
Watts and Scott, Recent developments in the classification and assessment of vasculitis, Best Pract Res Clin Rheumatol. 2009;23(3):429
Weber, B., Saurer, L., Schenk, M., Dickgreber, N., and Mueller, C. (2011). CX3CR1 defines functionally distinct intestinal mononuclear phagocyte subsets which maintain their respective functions during homeostatic and inflammatory conditions. Eur J Immunol 41, 773-779.
Worbs, T., Bode, U., Yan, S., Hoffmann, M.W., Hintzen, G., Bernhardt, G., Forster, R., and Pabst, O. (2006). Oral tolerance originates in the intestinal immune system and relies on antigen carriage by dendritic cells. J Exp Med 203, 519-527.
Zigmond, E., Halpern, Z., Elinav, E., Brazowski, E., Jung, S., and Varol, C. (2011). Utilization of murine colonoscopy for orthotopic implantation of colorectal cancer. PLoS One 6, e28858.

## Claims

1. An agent capable of binding to the C-C chemokine receptor type 2 (CCR2) and inducing the depletion of CCR2-positive monocytes in a subject for use in the treatment and/or prevention of an inflammatory disease of the gastrointestinal system.

2. A method of treating and/or preventing an inflammatory disease of the gastrointestinal system comprising administering a pharmaceutically effective amount of an agent capable of binding to CCR2 and inducing the depletion of CCR2-positive monocytes in a subject to a subject in need thereof.

3. The agent of claim 1 or the method of claim 2, wherein the agent capable of binding to CCR2 and inducing the depletion of CCR2-positive monocytes in a subject is an antibody.

4. The agent of any one of claims 1 or 3, having admixed thereto or associated in a separate container one or more agents selected from the group consisting of antibiotic compounds, 5-aminosalicylates (5-ASA), steroids, immunomodulators, preferably TNF-alpha blockers, azathioprine and calcineurin inhibitors.

5. The method of any one of claims 2 or 3, further comprising the administration of one or more agents selected from the group consisting of antibiotic compounds, 5-aminosalicylates (5-ASA), steroids, immunomodulators, preferably TNF-alpha blockers, azathioprine and calcineurin inhibitors.

6. A pharmaceutical composition comprising the agent of any one of claims 1, 3 and 4, and optionally a pharmaceutically acceptable carrier.

7. The agent of any one of claims 1, 3 and 4, or the method of any one of claims 2, 3 and 6, wherein the inflammatory disease of the gastrointestinal system is selected from the group consisting of Crohn's disease, ulcerative colitis, collagenous colitis, lymphocytic colitis, diversion colitis, Behçet's disease, celiac disease, Whipple's disease, vasculitis affecting the intestine and indeterminate colitis.
